# EUROPEAN PATENT APPLICATION

(11) **EP 3 989 235 A1**
(43) Date of publication of application: **27.04.2022**
(21) Application number: 20826101.6
(22) Date of filing: 18.06.2020
(51) Int. Cl.: G16H 30/40, G01N 33/543

(54) **PROGRAM, TESTING DEVICE, INFORMATION PROCESSING DEVICE, AND INFORMATION PROCESSING METHOD**

(30) Priority: 19.06.2019 JP 2019113737
(71) Applicant: H.U. Group Research Institute G.K., Tokyo 192-0031 (JP)
(72) Inventor: FUTADA, Haruhiko, Tokyo 163-0408 (JP)
(74) Representative: Thibon, Laurent
(86) International application number: PCT/JP2020/023882
(87) International publication number: WO 2020/256041

(57) **Abstract**

Provided is a program or the like capable of reducing a work burden of a medical service worker or the like, in a testing using a testing kit for detecting a specimen by using an immunochromatography method. A testing device acquires a shot image obtained by shooting a testing kit for testing a specimen by using an immunochromatography method. The testing device inputs the acquired shot image to a learned model for result discrimination that is deep-learned to output information relevant to a testing result of the testing kit in the shot image when the shot image of the testing kit is inputted. Then, the testing device determines the testing result of the testing kit in the acquired shot image, on the basis of the information outputted from the learned model for result discrimination and outputs the determined testing result.

## Description

### [Technical Field]

The present disclosure relates to a program, a testing device, an information processing apparatus, and an information processing method.

### [Background Art]

Recently, in a medical workplace, a clinical testing referred to as a point of care testing (POCT) that is performed by a medical service worker in the vicinity of a subject has been widespread. In POCT, for example, a testing kit for testing the presence or absence of a testing target by using an immunochromatography method that is an immunoassay method using a capillary action has been used (for example, refer to Patent Document 1). Various testings can be performed by preparing the testing kit using the immunochromatography method for each testing target such as influenza virus, norovirus, pneumococcus, and adenovirus, and using the testing kit according to an item to be tested (the testing target). In the testing kit using the immunochromatography method, the presence or absence (the positive or negative) of the testing target is determined by dropping a specimen (a test body) sampled from the subject into a predetermined dropping region, and visually checking the presence or absence of a determination line after a predetermined testing time elapses.

### [Prior Art Document]

### [Patent Document]

[Patent Document 1] Japanese Patent Application Laid-Open No. 2019-45286

### [Summary of Invention]

### [Problems to be Solved by Invention]

The testing kit as described above is sold by a plurality of manufacturers, a usage, a time required for the testing, or the like is different for each of the testing kits, and in a case where the testing is performed in a wrong usage, an accurate testing result may not be obtained. In particular, in a case where there are a plurality of testing targets (test bodies sampled from a subject), such as an outbreak of influenza, it is necessary to promptly obtain accurate testing results with respect to the plurality of testing targets, which is a great work burden to the medical service worker performing the testing.

The present disclosure has been made in consideration of such circumstances, and an object thereof is to provide a program or the like capable of reducing a work burden of a medical service worker or the like performing a testing.

### [Means for Solving Problems]

A program according to one aspect of the present disclosure causes a computer to execute processing of: acquiring an image obtained by shooting a testing kit for testing a specimen by using an immunochromatography method; inputting the acquired image to a learned model for result discrimination that is deep-learned to output information relevant to a testing result of the testing kit in the shot image when the shot image of the testing kit is inputted; determining the testing result of the testing kit in the acquired image, on the basis of the information outputted from the learned model for result discrimination; and outputting the determined testing result.

### [Effects of Invention]

According to the present disclosure, it is possible to reduce a work burden of a tester such as a medical service worker, in a testing using a testing kit for detecting a specimen by using an immunochromatography method.

### [Brief Description of Drawings]

FIG. 1A is a schematic view illustrating an appearance example of a testing device.
FIG. 1B is a schematic view illustrating an appearance example of a testing kit.
FIG. 2 is a block diagram illustrating a configuration example of the testing device.
FIG. 3A is a schematic view illustrating a configuration example of a DB stored in the testing device.
FIG. 3B is a schematic view illustrating a configuration example of the DB stored in the testing device.
FIG. 4 is a schematic view illustrating a configuration example of a class discriminant model.
FIG. 5 is a flowchart illustrating an example of a determination processing procedure of the testing device.
FIG. 6 is a flowchart illustrating an example of the determination processing procedure of the testing device.
FIG. 7A is a schematic view illustrating a screen example.
FIG. 7B is a schematic view illustrating a screen example.
FIG. 8 is a schematic view illustrating a configuration example of a testing system of Embodiment 2.
FIG. 9 is a block diagram illustrating a configuration example of the testing system of Embodiment 2.
FIG. 10 is a flowchart illustrating an example of a determination processing procedure of a server of Embodiment 2.
FIG. 11A is a schematic view illustrating a screen example.
FIG. 11B is a schematic view illustrating a screen example.
FIG. 11C is a schematic view illustrating a screen example.
FIG. 11D is a schematic view illustrating a screen example.
FIG. 12 is a flowchart illustrating an example of a determination processing procedure of a server of Embodiment 3.
FIG. 13A is a schematic view illustrating a screen example.
FIG. 13B is a schematic view illustrating a screen example.
FIG. 14 is a flowchart illustrating an example of a determination processing procedure of a server of Embodiment 4.
FIG. 15 is a flowchart illustrating an example of a determination processing procedure of a testing device of Embodiment 5.
FIG. 16 is a flowchart illustrating an example of a determination processing procedure of a server of Embodiment 6.
FIG. 17 is a flowchart illustrating an example of a determination processing procedure of a server of Embodiment 7.
FIG. 18 is a flowchart illustrating an example of a determination processing procedure of a testing device of Embodiment 8.
FIG. 19 is a flowchart illustrating an example of the determination processing procedure of the testing device of Embodiment 8.

### [Mode for Carrying out Invention]

Hereinafter, a program, a testing device, an information processing apparatus, and an information processing method of the present disclosure will be described in detail, on the basis of the drawings illustrating embodiments thereof.

### (Embodiment 1)

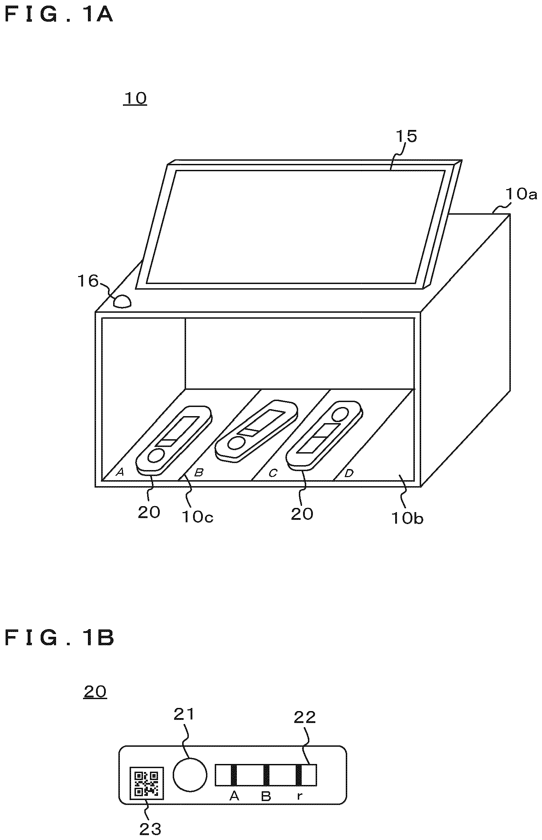

A testing device determining a testing result of a testing kit using an immunochromatography method by using a neural network (a learned model) will be described. FIG. 1A is a schematic view illustrating an appearance example of the testing device, and FIG. 1B is a schematic view illustrating an appearance example of the testing kit. Specifically, FIG. 1A is a perspective view of the testing device, and FIG. 1B is a top view of the testing kit. A testing device 10, for example, is a device to be used by being installed in a medical institution, and includes a storage portion 10a that is a rectangular housing, and a display unit 15 and a notification light 16, which are provided on the upper surface of the storage portion 10a. In the storage portion 10a, one lateral surface is opened, and a testing kit 20 is inserted into the storage portion 10a from an opening portion and taken out to the outside of the storage portion 10a.

The bottom surface in the storage portion 10a is a placing surface 10b (a placing table) on which a plurality of testing kits 20 can be placed in parallel, and areas A to D in which the testing kits 20 are respectively placed are parted by a parting line 10c. In the example illustrated in FIG. 1A, four testing kits 20 can be placed on the placing surface 10b, and the areas A to D in which the testing kits 20 are respectively placed are provided to extend to a depth direction in the storage portion 10a from the opening portion. Note that, the number of testing kits 20 to be placed on the placing surface 10b is not limited to 4, and the extending direction of each of the areas in which the testing kit 20 is placed is not limited to the direction in FIG. 1A. In addition, on the lateral surface of the storage portion 10a, which is opened, a lid portion that is capable of opening and closing the opening portion may be provided. The parting line 10c may be a line segment drawn on the placing surface 10b, or may be a convex portion or a concave portion, and the parting line 10c is not limited insofar as the parting line is capable of partitioning each of the areas A to D when a tester performing a testing places the testing kit 20.

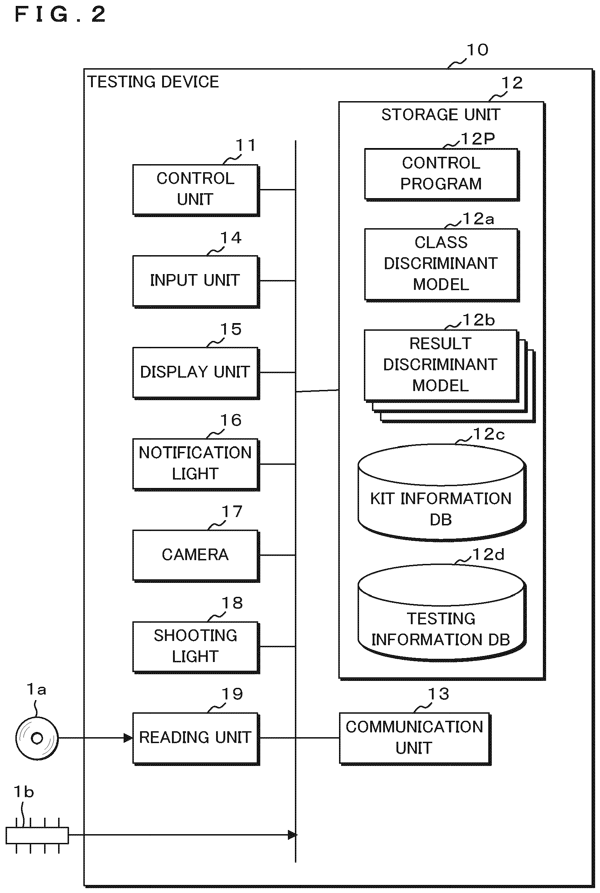

In addition, on the upper surface in the storage portion 10a, a camera 17 and a shooting light 18 (refer to FIG. 2) for shooting the testing kit 20 placed in each of the areas A to D are provided. In this embodiment, the testing kits 20 placed in the areas A to D are shot by using one camera 17 and one shooting light 18, but the camera 17 and the shooting light 18 may be provided for each of the areas A to D.

As illustrated in FIG. 1A and FIG. 1B, the testing kit 20 is configured approximately in the shape of a rectangular plate, and includes a dropping region 21 and a determination region 22 on one surface. The dropping region 21 is a region in which a specimen (a test body) sampled from a subject is dropped, and the determination region 22 is a region indicating a testing result (whether or not a testing target is included) with respect to the specimen dropped in the dropping region 21. The specimen, for example, can be a nasal cavity swab, a pharynx swab, nasal discharge, urine, saliva, blood, and the like of the subject. In the determination region 22, an antibody (an antibody for a testing) to react with (to be bonded to) the testing target is applied into the shape of a line, and in a case where the testing target reacts with the antibody for a testing, the determination region 22 produces a blue color, a red-purple color, or the like. Accordingly, in a case where the testing target is included in the specimen, the testing target included in the specimen reacts with the antibody for a testing and produces a color, and a determination line appears in the determination region 22. FIG. 1B illustrates an example of the testing kit 20 for influenza virus, and in the testing kit 20 illustrated in FIG. 1B, an influenza A antibody for a testing and an influenza B antibody for a testing are applied. In such a testing kit 20, an influenza A testing and an influenza B testing can be simultaneously performed. In addition, in the determination region 22, in a position away from the dropping region 21, an antibody (an antibody for determining an end) to react with (to be bonded to) a labeled antibody is applied into the shape of a line, and in a case where the labeled antibody reacts with the antibody for determining an end, the determination region 22 produces a blue color, a red-purple color, or the like. Accordingly, for example, in a case where the labeled antibody applied in the dropping region 21 is moved in the determination region 22 together with the specimen and reaches the position in which the antibody for determining an end is applied, the labeled antibody reacts with the antibody for determining an end and produces a color, and a testing end line (a control line) appears in the determination region 22. In the determination region 22 illustrated in FIG. 1B, an influenza A determination line, an influenza B determination line, and the testing end line appear in order of proximity to the dropping region 21, but the present disclosure is not limited to such a configuration. In a case where the testing end line is in the farthest position from the dropping region 21, the positions of two determination lines may be reversed. In addition, the testing kit 20 may be configured such that only the influenza A determination line appears, or may be configured such that only the influenza B determination line appears.

In the testing kit 20, a code 23 (code information) associated with the subject is added to the surface on which the dropping region 21 and the determination region 22 are provided. In FIG. 1B, the code 23 using a QR code (Registered Trademark) is attached, but the present disclosure is not limited thereto. For example, a seal in which a patient registration card number (the code 23) in the medical institution, a bar code associated with the patient registration card number (the code 23), or the like is described may be stuck, or the patient registration card number (the code 23) may be filled in by handwriting.

The testing kit 20 having the configuration described above is used in the testing in a state where the surface on which the dropping region 21 and the determination region 22 are provided is placed as the upper surface. Accordingly, the surface on which the dropping region 21 and the determination region 22 are provided is set as the upper surface. In a case where the testing result of the testing kit 20 is determined by the testing device 10, the testing kit 20 is placed in any of the areas A to D on the placing surface 10b of the testing device 10 such that the upper surface is on the upper side. A position in which the testing kit 20 is placed is not limited insofar as the position is in each of the areas A to D, and a direction in which the testing kit 20 is placed (a direction at the time of placing the testing kit 20) may be any direction.

FIG. 2 is a block diagram illustrating a configuration example of the testing device 10. The testing device 10 is configured by using a personal computer, a server computer, or the like, and includes a control unit 11, a storage unit 12, a communication unit 13, an input unit 14, a display unit 15, a notification light 16, a camera 17, a shooting light 18, a reading unit 19, and the like, in which such units are connected to each other through a bus. The control unit 11 includes one or a plurality of processors such as a central processing unit (CPU), a micro-processing unit (MPU), or a graphics processing unit (GPU). The control unit 11 allows the testing device 10 to perform various information processings, control processings, or the like, to be performed by the testing device of the present disclosure, by suitably executing a control program 12P stored in the storage unit 12.

The storage unit 12 includes a random access memory (RAM), a flash memory, a hard disk, a solid state drive (SSD), and the like. The storage unit 12 stores in advance the control program 12P executed by the control unit 11, various data items required for executing the control program 12P, and the like. In addition, the storage unit 12 temporarily stores data or the like that is generated when the control unit 11 executes the control program 12P. In addition, the storage unit 12, for example, stores a class discriminant model 12a (a learned model for class discrimination) and a result discriminant model 12b (a learned model for result discrimination), which are a learned model constructed by deep learning processing. In a case where image data (a shot image) obtained by shooting the upper surface of the testing kit 20 as illustrated in FIG. 1B is inputted, the class discriminant model 12a is a learned model that is learned to output information (information relevant to the class) indicating which class registered in advance the class of the testing kit 20 in the shot image is. In a case where the image data (the shot image) obtained by shooting the upper surface of the testing kit 20 is inputted, the result discriminant model 12b is a learned model that is learned to output information (information relevant to a testing result) indicating that the testing result of the testing kit 20 in the shot image is positive or negative. The result discriminant model 12b is provided for each class (type) of the testing kit 20 that is a discriminant target of the class discriminant model 12a. The learned model performs predetermined calculation with respect to an input value and outputs a calculation result, and in the storage unit 12, data such as a coefficient or a threshold value of a function for defining the calculation is stored as each of the class discriminant model 12a and the result discriminant model 12b. In addition, the storage unit 12 stores a kit information database (DB) 12c and a testing information DB 12d, described below. Note that, the kit information DB 12c and the testing information DB 12d may be stored in an external storage device that is connected to the testing device 10, or may be stored in an external storage device to which the testing device 10 is capable of performing communication through a network.

The communication unit 13 is an interface to be connected to a network such as the Internet or a local area network (LAN) by wired communication or wireless communication, and performs the transmission and reception of information with respect to an external device through the network. In this embodiment, the communication unit 13 is configured to be capable of performing communication with respect to an electronic health record system in the medical institution through the network. The input unit 14 receives manipulation input of a user (the tester), and transmits a control signal corresponding to the manipulation content to the control unit 11. The display unit 15 is a liquid crystal display, an organic EL display, or the like, and displays various information items, in accordance with an instruction from the control unit 11. Note that, the input unit 14 and the display unit 15 may be a touch panel in which the input unit 14 and the display unit 15 are integrally configured.

The notification light 16 is a light emitting diode (LED) light, a rotating lamp, or the like, and notifies a testing situation of the testing device 10 to the tester by lighting or blinking in accordance with an instruction from the control unit 11. In this embodiment, one notification light 16 is provided, but the notification light 16 may be provided for each of the areas A to D on the placing surface 10b. In addition, a sound output device such as a buzzer or a speaker may be provided instead of the notification light 16 to notify the testing situation of the testing device 10 to the tester by a sound.

The camera 17 (a shooting unit) includes a lens, an imaging element, and the like, and acquires image data of a subject image through the lens. The camera 17 performs shooting, in accordance with an instruction from the control unit 11, and sequentially transmits the acquired image data (the shot image) to the control unit 11. The shooting light 18, for example, is a LED light, and is turned on, in accordance with an instruction from the control unit 11. Note that, the control unit 11 transmits a lighting instruction to the shooting light 18 and transmits a shooting execution instruction to the camera 17, at a shooting timing. Accordingly, the shooting can be performed by the camera 17 in a state where the shooting light 18 is turned on, and an excellent shot image can be obtained in which the testing kit 20 placed on the placing surface 10b is accurately shot. Note that, the camera 17 and the shooting light 18 may be externally connected to the testing device 10, in addition to a configuration in which the camera 17 and the shooting light 18 is built in the testing device 10. In this case, the testing device 10 includes a connection portion to which an external camera and an external shooting light can be connected, the operation of the external camera and the external shooting light is controlled through the connection portion, and image data shot by the external camera is acquired through the connection portion.

The reading unit 19 reads information stored in a portable storage medium 1a including a compact disc (CD)-ROM, a digital versatile disc (DVD)-ROM, and a universal serial bus (USB) memory. The control unit 11 may read the program and the data to be stored in the storage unit 12, for example, from the portable storage medium 1a through the reading unit 19 and store them into the storage unit 12. In addition, the control unit 11 may download the program and the data to be stored in the storage unit 12 from the external device through the communication unit 13 and the network and store them into
the storage unit 12. Further, the program and the data may be stored in a semiconductor memory 1b, and the control unit 11 may read out the program and the data from the semiconductor memory 1b.

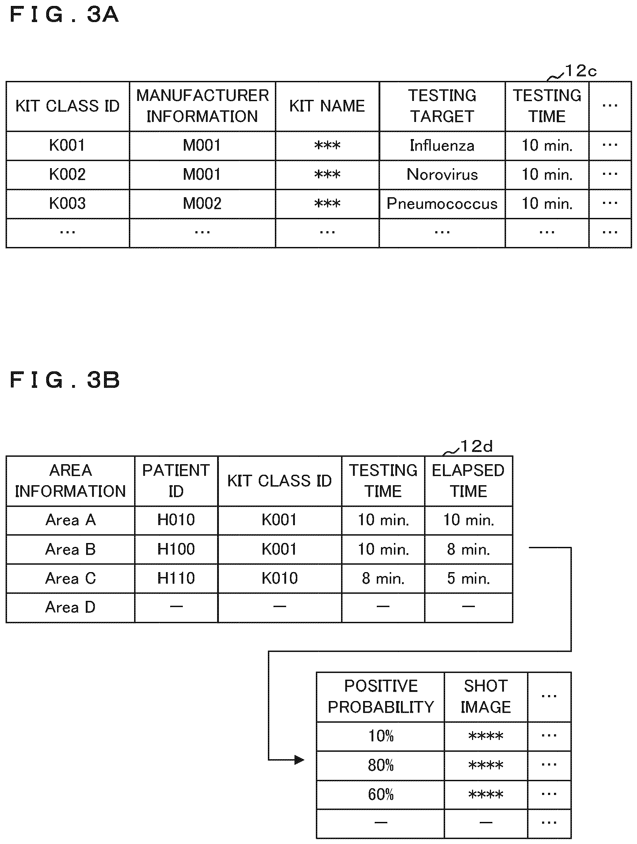

FIG. 3A and FIG. 3B are schematic views illustrating a configuration example of the DBs 12c and 12d stored in the testing device 10. FIG. 3A illustrates the kit information DB 12c, and FIG. 3B illustrates the testing information DB 12d. The kit information DB 12c stores information relevant to the testing kit 20 that is capable of performing determination processing of a testing result of the testing device 10. The kit information DB 12c illustrated in FIG. 3A includes a kit class ID column, a manufacturer information column, a kit name column, a testing target column, a testing time column, and the like. The kit class ID column stores identification information assigned to each class (type) of testing kit 20. The manufacturer information column stores information relevant to a manufacturer manufacturing or selling each of the testing kits 20, in association with the kit class ID, and the kit name column stores a name, a given name, or the like applied to each of the testing kits 20, in association with the kit class ID. The testing target column stores information relevant to a target that can be tested in each of the testing kits 20, in association with the kit class ID, and the testing time column stores a time required for each of the testing kits 20 to perform the testing, in association with the kit class ID. In a case where the control unit 11 acquires information of new type of testing kit 20 through the input unit 14 or the communication unit 13, the kit class ID stored in the kit information DB 12c is stored by being issued from the control unit 11. The information other than the kit class ID stored in the kit information DB 12c is added by the control unit 11 whenever the control unit 11 acquires an additional instruction through the input unit 14 or the communication unit 13. The stored content of the kit information DB 12c is not limited to the example illustrated in FIG. 3A, and various information items relevant to the testing kit 20 may be stored. For example, the use frequency, the stock quantity, or the like of each of the testing kits 20 may be stored in the kit information DB 12c.

The testing information DB 12d stores information relevant to a determination situation of the testing result of the testing device 10. The testing information DB 12d illustrated in FIG. 3B includes an area information column, a patient ID column, a kit class ID column, a testing time column, an elapsed time column, a positive probability column, a shot image column, and the like. The area information column stores identification information of the place areas A to D provided on the placing surface 10b of the testing device 10. The patient ID column stores identification information (for example, the patient registration card number in the medical institution) of a patient (the subject) corresponding to the testing kit 20 placed in each of the areas A to D, in association with the area information. The kit class ID column stores identification information of the type of testing kit 20 placed in each of the areas A to D, in association with the area information, and the testing time column stores a time required for the testing kit 20 placed in each of the areas A to D to perform the testing, in association with the area information. The elapsed time column stores an elapsed time after the testing kit 20 is placed in each of the areas A to D, in association with the area information. The positive probability column stores a probability that the testing result of the testing kit 20 placed in each of the areas A to D is positive, in association with the area information, and the shot image column stores the shot image of the testing kit 20 placed in each of the areas A to D, in association with the area information. Note that, the data of the shot image may be stored not only in the testing information DB 12d, but also in a predetermined region of the storage unit 12 or the external storage device connected to the testing device 10. In this case, the shot image column stores information (for example, a file name indicating a storage location of the data) for reading out the data of the shot image. The area information stored in the testing information DB 12d is stored in advance. The patient ID stored in the testing information DB 12d is stored by the control unit 11 whenever the control unit 11 specifies the identification information (the patient ID) of the patient corresponding to the testing kit 20 in the shot image, on the basis of the shot image of the camera 17. The kit class ID and the testing time, stored in the testing information DB 12d, is stored by being read out from the kit information DB 12c by the control unit 11 whenever the control unit 11 discriminates the class of the testing kit 20 in the shot image, on the basis of shot image of the camera 17. As the elapsed time stored in the testing information DB 12d, in a case where the control unit 11 detects that the testing kit 20 is placed in each of the areas A to D, on the basis of the shot image of the camera 17, an elapsed time from the detection is stored by the control unit 11. The positive probability stored in the testing information DB 12d is stored by the control unit 11 whenever the control unit 11 acquires a probability indicating that the testing result of the testing kit 20 in the shot image is positive, on the basis of the shot image of the camera 17. The shot image stored in the testing information DB 12d is stored and updated by the control unit 11 whenever the shot image is acquired by the camera 17. Note that, the shot image may be sequentially stored together with a shooting date or a shooting timing (for example, an elapsed time from a testing start), or only the newest shot image may be stored. The stored content of the testing information DB 12d is not limited to the example illustrated in FIG. 3B, and various information items relevant to each of the areas A to D on the placing surface 10b, the information relevant to the testing kit 20 placed in each of the areas A to D, or the like may be stored. In addition, the remaining determination time until the testing time expires may be stored instead of the elapsed time.

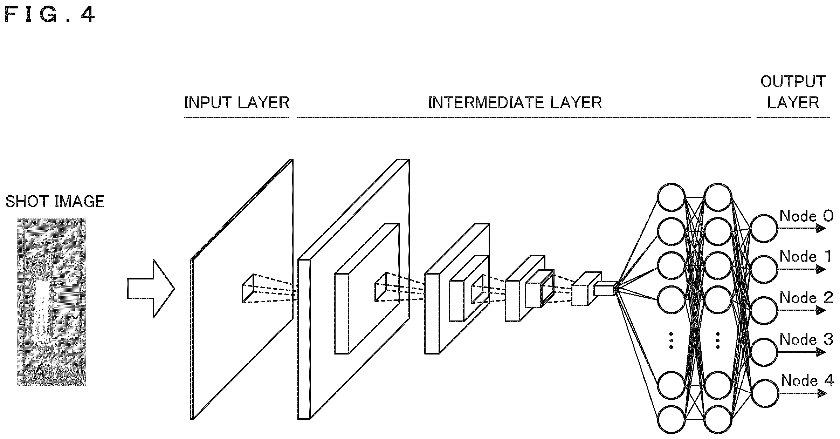

FIG. 4 is a schematic view illustrating a configuration example of the class discriminant model 12a. The class discriminant model 12a of this embodiment, for example, is configured as a convolution neural network (CNN) model, as illustrated in FIG. 4. The class discriminant model 12a may be configured as various learning models such as region-based CNN (R-CNN) and you only look once (YOLO), in addition to the CNN model. The class discriminant model 12a illustrated in FIG. 4 includes an input layer, an intermediate layer, and an output layer. The intermediate layer includes a convolution layer, a pooling layer, and a fully connected layer. In the class discriminant model 12a of this embodiment, the shot image (the image data) of the upper surface of the testing kit 20 is inputted through the input layer. Each pixel in the shot image of the testing kit 20 is inputted to each node of the input layer, and the input shot image of the testing kit 20 is inputted to the intermediate layer through each of the nodes of the input layer. The shot image of the testing kit 20 inputted to the intermediate layer is subjected to filter processing or the like in the convolution layer such that a feature amount of the image is extracted and a feature map is generated, and compressed in the pooling layer such that an information amount is reduced. A plurality of convolution layers and a plurality of pooling layers are repeatedly provided, and the feature map generated by the plurality of convolution layers and the plurality of pooling layers is inputted to the fully connected layer. A plurality of (in FIG. 4, two) fully connected layers are provided, an output value of the node of each of the layers is calculated by using various functions, threshold values, or the like, on the basis of the input feature map, and the calculated output value is sequentially inputted to the node of the subsequent layer. The fully connected layer sequentially inputs the output value of the node of each of the layers to the node of the subsequent layer such that each output value is finally applied to each node of the output layer. The number of layers of each of the convolution layer, the pooling layer, and the fully connected layer is not limited to the example illustrated in FIG. 4.

The class discriminant model 12a of this embodiment is configured to discriminate the class of five types of testing kits 20. Accordingly, in the class discriminant model 12a, the output layer includes five nodes 0 to 4, in which the node 0 outputs a probability that the testing kit 20 in the input shot image is to be discriminated as a first class, the node 1 outputs a probability that the testing kit 20 in the input shot image is to be discriminated as a second class, the node 2 outputs a probability that the testing kit 20 in the input shot image is to be discriminated as a third class, the node 3 outputs a probability that the testing kit 20 in the input shot image is to be discriminated as a fourth class, and the node 4 outputs a probability that the testing kit 20 in the input shot image is to be discriminated as a fifth class. Note that, an output node that outputs a probability the testing kit 20 in the input shot image is to be determined as a class other than the class of the discriminant target or the discrimination is to be determined as impracticable may be provided. The output value of each of the nodes of the output layer, for example, is a value of 0 to 1.0, and the total probability outputted from each of the five nodes is 1.0 (100%).

The class discriminant model 12a is learned by using teacher data including the shot image of the upper surface of the testing kit 20, and information indicating the class of the testing kit 20 in the shot image or information (a ground truth label) indicating that the testing kit 20 is not included in the shot image. In a case where the shot image included in the teacher data is inputted, the class discriminant model 12a is learned such that an output value from an output node corresponding to the ground truth label included in the teacher data is close to 1.0, and an output value from the other output node is close to 0. In learning processing, the class discriminant model 12a optimizes data such as coefficients or threshold values of various functions for defining predetermined calculation that is performed with respect to the input value. Accordingly, in a case where the shot image is inputted, the learned class discriminant model 12a that is learned to output the information indicating the class of the testing kit 20 in the shot image is obtained. Note that, the learning of the class discriminant model 12a, for example, is performed by another learning device. The learned class discriminant model 12a that is generated by being learned with the learning device, for example, is downloaded into the testing device 10 from the learning device through the network or through the portable storage medium 1a and stored in the storage unit 12.

As with the class discriminant model 12a, the result discriminant model 12b of this embodiment, for example, is also configured as the CNN model as illustrated in FIG. 4. Similarly, the result discriminant model 12b may be configured as various learning models such as R-CNN and YOLO. As with the class discriminant model 12a, in the result discriminant model 12b of this embodiment, the shot image of the upper surface of the testing kit 20 is inputted through the input layer. Note that, the result discriminant model 12b is provided for each class of the testing kit 20, and for example, the result discriminant model 12b corresponding to the testing kit 20 for the influenza A testing and the influenza B testing is configured to discriminate any of four patterns in which the testing result of the testing kit 20 in the shot image is positive in both of the influenza A testing and the influenza B testing, negative in both of the influenza A testing and the influenza B testing, positive only in the influenza A testing, and positive only in the influenza B testing. Accordingly, the output layer of the result discriminant model 12b includes four nodes 0 to 3, in which for example, the node 0 outputs a probability that the testing result of the testing kit 20 in the input shot image is to be discriminated as a positive in both of the influenza A testing and the influenza B testing, the node 1 outputs a probability that the testing result of the testing kit 20 in the input shot image is to be discriminated as a negative in both of the influenza A testing and the influenza B testing, the node 2 outputs a probability that the testing result of the testing kit 20 in the input shot image is to be discriminated as a positive only in the influenza A testing, and the node 3 outputs a probability that the testing result of the testing kit 20 in the input shot image is to be discriminated as a negative only in the influenza B testing. The output value of each of the nodes of the output layer, for example, is a value of 0 to 1.0, and the total probability outputted from each of the four nodes is 1.0 (100%). Note that, an output node that outputs a probability that the discrimination is to be determined as impracticable may be provided.

The result discriminant model 12b is learned by using teacher data including the shot image of the upper surface of the testing kit 20 and information (a ground truth label) indicating the testing result (for example, any of the four patterns) of the testing kit 20 in the shot image. In a case where the shot image included in the teacher data is inputted, the result discriminant model 12b is learned such that an output value from an output node corresponding to the ground truth label included in the teacher data is close to 1.0, and an output value from the other output node is close to 0. Accordingly, in a case where the shot image is inputted, the learned result discriminant model 12b that is learned to output the information indicating which of the four patterns the testing result of the testing kit 20 in the shot image is. Note that, the learning of the result discriminant model 12b, for example, is also performed by another learning device, and the learned result discriminant model 12b that is generated by the learning device, for example, is downloaded into the testing device 10 from the learning device through the network or through the portable storage medium 1a and stored in the storage unit 12.

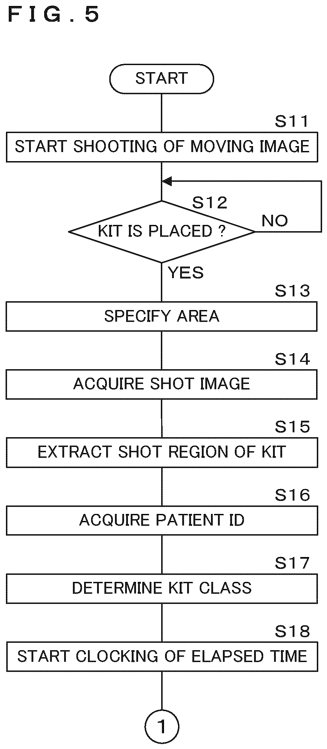

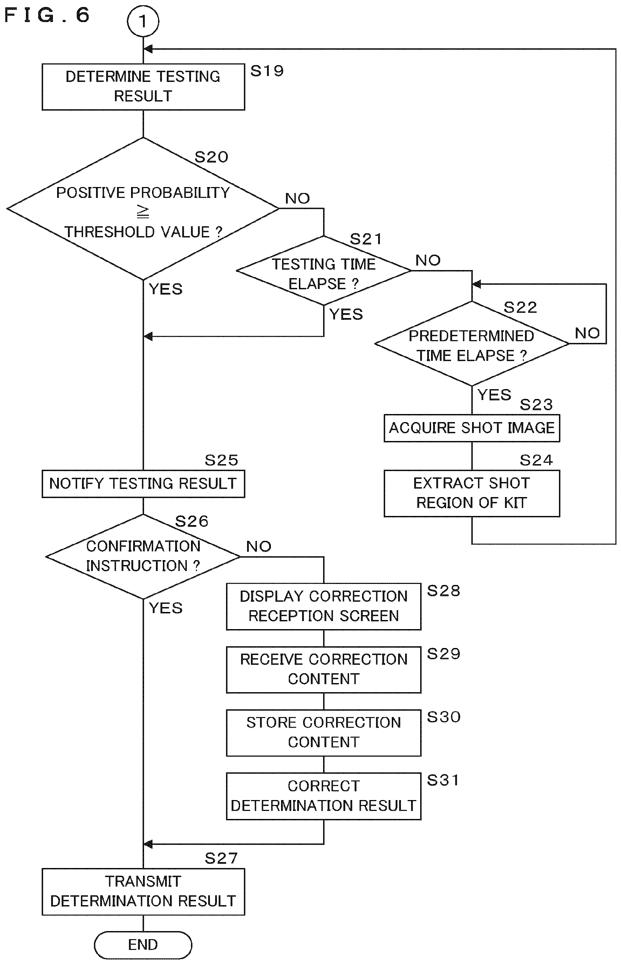

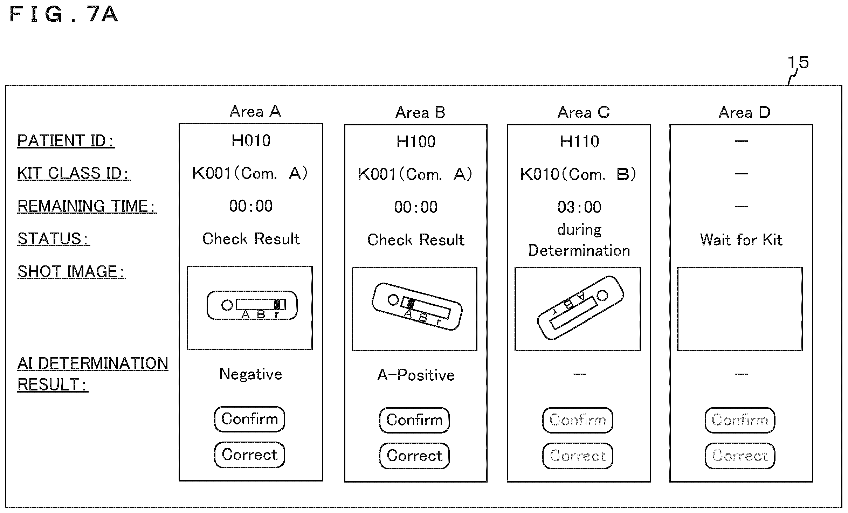

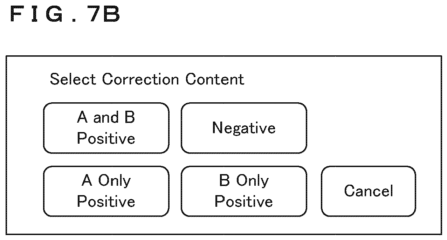

Hereinafter, processing will be described in which the testing device 10 of this embodiment determines the testing result of the testing kit 20. FIG. 5 and FIG. 6 are flowcharts illustrating an example of a determination processing procedure of the testing device 10, and FIG. 7A and FIG. 7B are schematic views illustrating a screen example. The following processing is executed by the control unit 11, in accordance with the control program 12P stored in the storage unit 12 of the testing device 10. Note that, in this embodiment, the following processing is attained by the control unit 11 executing the control program 12P, and a part of the processing may be attained by a dedicated hardware circuit.

In a case where the operation of the testing device 10 is started, the control unit 11 starts the shooting of a moving image using the camera 17 (S11). The control unit 11, for example, acquires 30 frames (30 sheets) of the image data (the shot image) for 1 second by the camera 17, and judges whether or not the testing kit 20 is placed in any of the areas A to D on the placing surface 10b, on the basis of the sequentially acquired shot images (S12). For example, the control unit 11 calculates a difference between the sequentially acquired shot images, and judges whether or not the testing kit 20 is placed, on the basis of the difference between the shot images. Specifically, in a case where the difference between the shot images is greater than or equal to a predetermined amount, it may be judged that the testing kit 20 is placed. In addition, for example, in a case where the shot image is inputted, the control unit 11 may judge whether or not the testing kit 20 is placed by using a learned model learned to output information indicating whether or not the testing kit 20 is shot in the shot image. In this case, the control unit 11 is capable of sequentially inputting the shot image of the camera 17 to the learned model, and judging whether or not the testing kit 20 is included in the shot image, that is, whether or not the testing kit 20 is placed, on the basis of the output information from the learned model.

In a case where it is judged that the testing kit 20 is not placed on the placing surface 10b (S12: NO), the control unit 11 continues the processing of judging whether or not the testing kit 20 is placed on the placing surface 10b, on the basis of the shot image that is sequentially acquired by the camera 17. In a case where it is judged that the testing kit 20 is placed in any of the areas A to D on the placing surface 10b (S12: YES), the control unit 11 specifies the areas A to D in which the testing kit 20 is placed (S13). For example, in the shot image shot by the camera 17, a region (a shooting range) corresponding to each of the areas A to D is registered in advance, and the control unit 11 judges to which of the areas A to D a region (a shooting range) in which the difference between the shot images is greater than or equal to the predetermined amount corresponds, and specifies the corresponding areas A to D. In addition, the control unit 11 may judge to which of the areas A to D a region (a shooting range) in which it is judged that the testing kit 20 is placed by using a learned model corresponds, and specify the corresponding areas A to D. In addition, characters A to D may be described in each of the areas A to D on the placing surface 10b, and the control unit 11 may read the characters A to D included in the region in which the difference between the shot images is greater than or equal to the predetermined amount, or the region in which it is judged that the testing kit 20 is placed by using the learned model, for example, by using an optical character reader (OCR), may specify the area in which the testing kit 20 is placed. Further, a manipulation button for instructing the start of the judgement processing with respect to each of the areas A to D may be provided in the input unit 14, and the fact that the testing kit 20 is placed and the areas A to D in which the testing kit 20 is placed may be specified in accordance with the input of the tester. According to such a configuration, in a case where the areas A to D in which the testing kit 20 is placed are not capable of being specified on the basis of the shot image, or in a case where the judgement processing is tried to be started early, the judgement processing can be reliably started. In a case where the areas A to D in which the testing kit 20 is placed are specified, the control unit 11 resets each information item corresponding to area information of the specified areas A to D, in the testing information DB 12d.

Next, the control unit 11 acquires the shot image on the placing surface 10b by using the camera 17 and the shooting light 18 (S14). Then, the control unit 11 (an extraction unit) extracts a shot region of the areas A to D specified in step S13, that is, a shot region of the testing kit 20 that is judged as being placed in step S12 from the acquired shot image (S15). For example, the control unit 11 extracts the shot region of each of the areas A to D, on the basis of the parting line 10c on the placing surface 10b, as with the shot image illustrated in FIG. 4. In a case where the shot region of the testing kit 20 is extracted, the control unit 11 stores the acquired shot region in the testing information DB 12d, as a shot image corresponding to the area information of the areas A to D specified in step S13. Then control unit 11 reads the code 23 described in the shot testing kit 20, on the basis of the shot image (the shot region of the testing kit 20) stored in the testing information DB 12d, and acquires the identification information (the patient ID) of the patient (S16). For example, in a case where the code 23 is a QR code, the control unit 11 analyzes the QR code in the shot image, and acquires the patient ID (for example, the patient registration card number in the medical institution). In addition, in a case where the code 23 is a handwritten patient ID, the control unit 11 acquires the handwritten patient ID by reading the handwritten patient ID in the shot image with an OCR. Further, the control unit 11 may acquire the patient ID by the input of the tester using the input unit 14. In a case where the patient ID is acquired, the control unit 11 stores the acquired patient ID in the testing information DB 12d, in association with the area information of the areas A to D specified in step S13.

Next, the control unit 11 determines the class of the testing kit 20 in the shot image, on the basis of the shot image (the shot region of the testing kit 20) stored in the testing information DB 12d (S17). In this embodiment, the control unit 11 determines the class of the testing kit 20 by using the class discriminant model 12a. Specifically, the control unit 11 inputs the shot image to the class discriminant model 12a, and determines the class of the testing kit 20 in the shot image, on the basis of the information (the output information from each of the output nodes) outputted from the class discriminant model 12a. For example, the control unit 11 specifies an output node having the maximum output value in the output nodes of the class discriminant model 12a, and specifies the class associated with the specified output node as the class of the testing kit 20 in the shot image. Note that, in a case where the code 23 of each of the testing kits 20 includes the information of the class of the testing kit 20, the control unit 11 may acquire the information of the class from the information of the code 23 read from the shot image. In addition, the control unit 11 may acquire the information of the class of the testing kit 20 by the input of the tester using the input unit 14. In a case where the class of the testing kit 20 is specified, the control unit 11 reads out the kit class ID of the specified class and the testing time from the kit information DB 12c to be stored in the testing information DB 12d, in association with the area information of the areas A to D specified in step S13.

Next, the control unit 11 starts the clocking of the elapsed time from the testing start in the testing kit 20 (S18). Note that, the control unit 11 stores the elapsed time to be clocked in the testing information DB 12d, in association with the area information of the areas A to D specified in step S13, for example, every predetermined time such as every 1 second or every 10 seconds. Next, the control unit 11 (a determination unit) specifies the result discriminant model 12b according to the class determined in step S17, determines the testing result of the testing kit 20 in the shot image (the shot region of the testing kit 20) stored in the testing information DB 12d by using the specified result discriminant model 12b (S19). Here, the control unit 11 inputs the shot image to the result discriminant model 12b, and determines the testing result of the testing kit 20 in the shot image, on the basis of the information (the output information from each of the output nodes) outputted from the result discriminant model 12b. Specifically, the control unit 11 specifies the output node having the maximum output value in the output nodes of the result discriminant model 12b, and acquires the output value (the maximum output value) from the specified output node in a case where the testing result associated with the specified output node is positive (in the case of the influenza testing kit 20, positive in both of the influenza A testing and the influenza B testing, positive only in the influenza A testing, or positive only in the influenza B testing). That is, the control unit 11 acquires a probability (a positive probability) that the testing result is to be discriminated as a positive. In a case where the positive probability is acquired, the control unit 11 stores the acquired positive probability in the testing information DB 12d, in association with the area information of the areas A to D specified in step S13. Note that, the control unit 11 may store a testing result corresponding to the positive probability (a positive result) in the testing information DB 12d, together with the positive probability. According to the processing described above, in a case where the testing kit 20 is placed in any of the areas A to D, the information relevant to the testing kit 20 (the kit class ID and the testing time), the information (the patient ID) of the patient corresponding to the testing kit 20, and the determination situation (the positive probability) of the testing result of the testing kit 20 are specified from the shot image of the testing kit 20 and stored in the testing information DB 12d.

The control unit 11 judges whether or not the positive probability (a determination probability) that is indicated by the testing result determined in step S19 is greater than or equal to a predetermined threshold value (S20). Here, the threshold value is a value that is capable of confirming that the testing result of the testing kit 20 in the shot image is positive, and for example, can be 80% (0.8). The threshold value is set in advance, and for example, stored in the storage unit 12. In a case where it is judged that the positive probability is less than the threshold value (S20: NO), the control unit 11 judges whether or not the elapsed time at the time point (the elapsed time from the testing start) exceeds the testing time (a time required for a testing) of the testing kit 20 (S21). Specifically, the control unit 11 judges whether or not the elapsed time stored in the testing information DB 12d reaches the testing time, and judges that the elapsed time exceeds the testing time (that is, the testing time elapses) in a case where the elapsed time reaches the testing time. In a case where it is judged that the elapsed time does not exceed the testing time (S21: NO), the control unit 11 waits until a predetermined time elapses from the immediate shooting of the testing kit 20. Here, the predetermined time is a time interval for executing the processing of determining the testing result of the testing kit 20 in the shot image by shooting the testing kit 20, and for example, may be 30 seconds or 1 minute.

The control unit 11 judges whether or not the predetermined time elapses from the immediate shooting of the testing kit 20 (S22), and waits in a case where it is judged that the predetermined time does not elapse (S22: NO). In a case where it is judged that the predetermined time elapses (S22: YES), the control unit 11 acquires the shot image of the testing kit 20 on the placing surface 10b by using the camera 17 and the shooting light 18 (S23). Then, the control unit 11 extracts the shot region of the areas A to D specified in step S13, that is, the shot region of the testing kit 20 that is a determination target, from the acquired shot image (S24), and the process returns to the processing of step S19. Note that, the control unit 11 stores the extracted shot region of the testing kit 20 in the testing information DB 12d, as the newest shot image corresponding to the area information of the areas A to D specified in step S13. Then, the control unit 11 determines the testing result of the testing kit 20 in the newest shot image stored in the testing information DB 12d by using he result discriminant model 12b (S19), and stores the obtained positive probability in the testing information DB 12d, as the newest positive probability corresponding to the area information of the areas A to D specified in step S13. Note that, when the process returns to the processing of step S19, the control unit 11 may acquire again the patient ID or the kit class corresponding to the testing kit 20, on the basis of the shot region of the testing kit 20 acquired in step S24. Then, the processing of step S19 may be performed after it is checked that the acquired patient ID or kit class is identical to the patient ID or the kit class (the kit class ID and the testing time) stored in the testing information DB 12d. In this case, it is possible to prevent testing results of each of the subjects from being mixed up.

The control unit 11 repeats the processing of steps S19 to S24 until the positive probability is greater than or equal to the predetermined threshold value, and thus, the testing kit 20 is shot whenever the predetermined time elapses (for example, every 1 minute), and the processing of determining the testing result of the testing kit 20 (acquiring the positive probability) is repeated on the basis of the shot image. In a case where it is judged that the positive probability is greater than or equal to the predetermined threshold value (S20: YES), the control unit 11 notifies the determined testing result (here, a positive testing result) by displaying the determined testing result on the display unit 15 (S25). On the other hand, in a case where it is judged that the testing time elapses in step S21 (S21: YES), that is, in a case where the testing time elapses before the positive probability is greater than or equal to the predetermined threshold value, the control unit 11 notifies the determined testing result (here, a negative testing result) by displaying the determined testing result on the display unit 15 (S25). Note that, in step S25, the control unit 11 may notify the end of the determination processing not only by the display of the testing result but also by the lighting or blinking of the notification light 16. In this case, even in a case where the tester is in a position separated from the testing device 10, the end of the determination processing can be grasped. In addition, in a case where the testing device 10 includes the sound output device, the end of the determination processing may be notified by the sound output of the sound output device.

FIG. 7A illustrates a testing result screen example displayed on the display unit 15, and the testing result screen displays the determination situation of the testing result of the testing kit 20 placed in each of the areas A to D. Specifically, for each of the areas A to D, the patient ID, the kit class ID, and the shot image, which are specified from the shot image of the testing kit 20, are displayed with respect to the testing kit 20 placed in each of the areas A to D. In addition, for each of the areas A to D, the remaining determination time (the remaining time) until the expiration of the testing time according to the class of the testing kit 20 and an execution situation (a status) of the determination processing are displayed, and in a case where the determination processing is ended, a determination result (an AI determination result) is displayed. Note that, when seen from the front side (a surface side on which the opening portion is provided) of the testing device 10, it is desirable that an arrangement position of each of the areas A to D on the placing surface 10b is coincident with a display position of the information relevant to each of the areas A to D (the information relevant to the testing kit 20 placed in each of the areas A to D) displayed on the testing result screen. In a case where the arrangement position is coincident with the display position, the testing kit 20 placed in each of the areas A to D is easily associated with the information of each of the areas A to D displayed on the testing result screen, and it is possible to prevent the testing results from being mixed up.

In a case where the patient ID, the kit class ID, and the shot image are stored in the testing information DB 12d, the control unit 11 displays each of the stored information items in a position corresponding to each of the areas A to D on the testing result screen. In addition, in a case where the elapsed time is stored (updated) in the testing information DB 12d, the control unit 11 calculates the remaining determination time, on the basis of the elapsed time after being updated, and the testing time of the testing kit 20, and displays the calculated remaining time in the position corresponding to each of the areas A to D on the testing result screen. Further, the control unit 11 displays "Wait for Kit" to the area in a vacant state, as status information of each of the areas A to D, updates the display to "during Determination" in a case where the determination processing is started, and updates the display to progress in a case where the determination processing is ended. Note that, in a case where the determination processing is ended, the control unit 11 displays the testing result (the AI determination result) determined in step S25, in the position corresponding to each of the areas A to D on the testing result screen. The status information is not limited to the example described above, and various information items indicating a progress situation of the determination processing, such as during the acquisition of the patient ID or during the discrimination of the kit class, may be used.

For each of the areas A to D, a confirm button for instructing the confirmation of the displayed determination result (the determined testing result) and correct button for instructing the correction (the modification) of the determination result are displayed on the testing result screen illustrated in FIG. 7A. The confirm button and the correct button are displayed to be capable of being manipulated in a case where the determination result is obtained, the confirm button and the correct button corresponding to the area in the vacant state or the area during the determination processing are displayed not to be capable of being manipulated. For the areas A to D to which the status of "Check Result" is displayed after the determination processing is ended, the tester checks the determination region 22 of the testing kit 20 placed in the areas A to D, and visually determines the testing result of the testing kit 20. Then, the tester manipulates the confirm button in a case where the own determination of the tester is coincident with the AI determination result, and manipulates the correct button in a case where the own determination is not coincident with the AI determination result. Accordingly, the control unit 11 receives a confirmation instruction for the displayed determination result in a case where the confirm button is manipulated on the testing result screen through the input unit 14, and receives a correction instruction for the displayed determination result in a case where the correct button is manipulated.

The control unit 11 judges whether or not the confirmation instruction for the determination result is received (S26), and in a case where it is judged that the confirmation instruction is received (S26: YES), the control unit 11 confirms the determination result to be transmitted to a predetermined device (S27), and ends the processing with respect to the area. For example, the control unit 11 associates the determination result with the patient ID of the patient to be transmitted to the electronic health record system from the communication unit 13 through the network. The electronic health record system adds the testing result of each of the patients, which is received from the testing device 10, to an electronic health record, and thus, the testing device 10 and the electronic health record system can be linked together. In a case where it is judged that the confirmation instruction is not received (S26: NO), that is, in a case where the correction instruction (a modification instruction) is received, the control unit 11 displays a correction reception screen as illustrated in FIG. 7B by superimposing the correction reception screen on the result display screen (S28). The correction reception screen, for example, as illustrated in FIG. 7B, displays each button (an influenza A and influenza B-both-positive button, a negative button, an influenza A-only-positive button, and an influenza B-only-positive button) for designating the testing result (a correction content) that is capable of being corrected with respect to the determined testing result and a cancel button for ending the correction processing. Note that, in the button for designating the correction content, a button for the determined testing result may be displayed not to be capable of being selected. That is, in the screen illustrated in FIG. 7A, in a case where the correct button with respect to the area A is manipulated, in the screen illustrated in FIG. 7B, the negative button may be displayed not to be capable of being selected. Accordingly, in a case where the determination result is corrected, the tester manipulates the button corresponding to the content to be corrected, and in a case where the determination result is not corrected, the tester manipulates the cancel button.

In the correction reception screen, in a case where the cancel button is manipulated through the input unit 14, the control unit 11 ends the display of the correction reception screen and returns to the display of the testing result screen. In the correction reception screen, in a case where the button corresponding to the correction content is manipulated through the input unit 14, the control unit 11 receives the correction content (a modification content) corresponding to the manipulated button (S29). That is, in a case where the influenza A and influenza B-both-positive button is manipulated, the control unit 11 receives a correction instruction for positive in both of the influenza A testing and the influenza B testing, and in a case where the influenza A-only-positive button is manipulated, the control unit 11 receives a correction instruction for positive only in the influenza A testing. The control unit 11 stores the received correction content in the storage unit 12, in association with the newest shot image stored in the testing information DB 12d and the class of the testing kit 20 (for example, the kit class ID) (S30). Note that, here, the stored correction content and shot image can be used in teacher data (relearning data) for relearning the result discriminant model 12b corresponding to the class of the kit class ID. The control unit 11 corrects the determination result to the received correction content (S31), confirms the determination result after being corrected to be transmitted to a predetermined device (for example, the electronic health record system) (S27), and ends the processing with respect to the area.

According to the processing described above, the testing device 10 of this embodiment automatically judges the fact that the testing kit 20 is placed and the areas A to D in which the testing kit 20 is placed in a case where the testing kit 20 is placed on the placing surface 10b. In addition, the testing device 10 automatically determines the patient ID corresponding to the testing kit 20 placed on the placing surface 10b, the class of the testing kit 20, and the testing result of the testing kit 20. Accordingly, in the testing using the testing kit 20, a work burden when the tester such as a medical service worker visually determines the testing result can be reduced. The testing kit 20 is sold by a plurality of manufacturers, a usage, a time required for the testing, or the like is different for each of the testing kits, and in a case where the testing is performed in a wrong usage, an accurate testing result may not be obtained. In contrast, by using the testing device 10 of this embodiment, the tester may place the testing kit 20 in any of the areas A to D on the placing surface 10b, and it is not necessary to accurately grasp the testing time or a determination method of the testing result for each of the testing kits 20, and thus, a burden on the tester can be reduced. In addition, in a case where the determination is manually performed, there is a possibility that the testing time, the determination method of the testing result, or the like is incorrect for each of the testing kits 20, but in the testing device 10 of this embodiment, such an error can be avoided, and the testing result of the testing kit 20 can be determined with a high accuracy. In this embodiment, testing results with respect to testing kits 20 of a plurality of manufacturers and a plurality of types of testing kits 20 can be determined by one testing device 10. Accordingly, it can be expected that the testing device 10 is particularly effective in a medical institution or the like using the testing kits 20 of the plurality of manufacturers and the plurality of types of testing kits 20.

In the testing device 10 of this embodiment, the testing kit 20 is shot every predetermined time, the testing result is determined on the basis of the obtained shot image, and for example, it is notified that the testing result is positive at a time point when the positive probability is greater than or equal to the predetermined threshold value (for example, 80%). Accordingly, in a case where the positive probability is greater than or equal to the predetermined threshold value before the testing time according to the testing kit 20 elapses, it is possible to notify the testing result early without waiting for the elapse of the testing time.
Accordingly, for example, even in a case where there are a plurality of testing targets, such as an outbreak of influenza, it is possible to accurately and promptly determine the testing result with respect to the plurality of testing targets. In particular, the testing result can be notified early to a subject having a positive testing result, and it can be expected that a waiting time in the medical institution is lessened.

In this embodiment, in a case where the testing result (the determination result) determined by the testing device 10 is incorrect, the determination result can be corrected through the testing result screen. Accordingly, in a case where the testing result automatically determined by the testing device 10 is incorrect, for example, the testing result can be corrected to a correct testing result inputted by the tester. In addition, the result discriminant model 12b can be relearned by using the corrected testing result and shot image, and in this case, a more accurate result discriminant model 12b can be attained. Note that, in a case where the result discriminant model 12b is relearned, for example, the learning device managed by a manufacturer collects the testing result corrected in each of the testing devices 10 and the shot image (teacher data for relearning), and the result discriminant model 12b is relearned by using the collected teacher data for relearning. Then, the relearned result discriminant model 12b is provided to the testing device 10, and thus, the result discriminant model 12b in each of the testing devices 10 can be updated, and a more accurate result discriminant model 12b can be used in each of the testing devices 10. In addition, the class of the testing kit 20 discriminated by using the class discriminant model 12a may be configured to be capable of being corrected in a case where the class is incorrect, and in this case, the class discriminant model 12a can also be relearned.

In this embodiment, the parting line 10c for clearly specifying the boundary between the areas A to D on the placing surface 10b of the testing device 10 is provided, but the parting line 10c is not essential. For example, in a case where the testing kit 20 is placed in an arbitrary position on the placing surface 10b, the testing device 10 specifies the placed position of each of the testing kits 20, and performs each processing in association with the specified position, and thus, is capable of performing the determination processing with respect to the testing kit 20 placed in the arbitrary position. For example, in a case where the class discriminant model 12a is configured as R-CNN, it is possible to attain the class discriminant model 12a that extracts the region of the testing kit 20 and discriminates the class of the testing kit 20, in the input shot image. In the case of using such a class discriminant model 12a, the parting line 10c may not be provided on the placing surface 10b. In addition, in this embodiment, the testing result of the testing kit 20 is discriminated by using one class discriminant model 12a and result discriminant models 12b (a plurality of result discriminant models 12b) for each of the classes, but this embodiment is not limited to such a configuration. For example, in a case where the shot image is inputted, the result discriminant model may be learned such that the testing result of the testing kit 20 is discriminated without discriminating the class of the testing kit 20 in the shot image. In this case, the testing result of the testing kit 20 in the input shot image can be determined by using one result discriminant model.

In this embodiment, since the subject corresponding to the testing kit 20 can be specified by reading the code 23 of the testing kit 20 placed on the placing surface 10b, the determined testing result can be linked with the electronic health record system. However, the testing device 10 may not have a configuration of specifying the subject. That is, in a case where the testing kit 20 is placed on the placing surface 10b, the testing device 10 may determine the class and the testing result of the testing kit 20 to be notified. In this case, each of the testing kits 20 and each of the subjects, for example, may be associated with each other by the tester.

In this embodiment, in a case where the positive probability discriminated by using the result discriminant model 12b is greater than or equal to the predetermined threshold value (greater than or equal to 80%), the determined result (the positive testing result) is confirmed, and displayed on the testing result screen, as the AI determination result. In addition, for example, in a case where the positive probability is greater than or equal to 90%, the determined result (the positive testing result) is confirmed, and displayed on the testing result screen, whereas in a case where the positive probability is greater than or equal to 80% and less than 90%, a message for urging the tester to check the determination result (for example, "Require Check") may be displayed together with the determined result (the positive testing result). By displaying such a message, in a case where a certainty factor with respect to the result (the positive probability) determined by using the result discriminant model 12b is less than a predetermined value, it is possible to urge the tester to check the result and provide a more accurate testing result to the subject.

### (Embodiment 2)

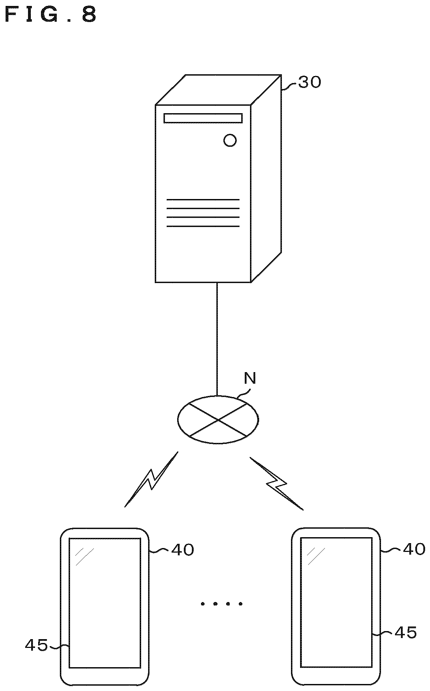

A testing system will be described in which the shot image of the testing kit 20 is acquired by using a user terminal, and a server (an information processing apparatus) in which the shot image is acquired from the user terminal determines the testing result of the testing kit 20. FIG. 8 is a schematic view illustrating a configuration example of the testing system of Embodiment 2. The testing system of this embodiment includes a plurality of user terminals 40, and a server 30 that acquires the shot image of the testing kit 20 from the user terminal 40, determines the testing result of the testing kit 20, on the basis of the shot image, and notifies the testing result to the user terminal 40. The server 30 and each of the user terminals 40 are configured to be capable of being connected to a network N such as the Internet, and perform the transmission and reception of information through the network N.

The server 30 is an information processing apparatus that is managed by an operator or the like operating the testing system of this embodiment, and is configured as a server computer, a personal computer, or the like.
A plurality of servers 30 may be provided, or the servers 30 may be attained by a plurality of virtual machines provided in one server or attained by using a cloud server. The user terminal 40 is a tablet terminal, a smart phone, a personal computer, or the like. In this embodiment, the user terminal 40 performs various information processings such as processing of shooting the testing kit 20, in accordance with the manipulation of the user and processing of transmitting the obtained shot image to the server 30. In addition, the server 30 performs various information processings such as processing of determining the testing result of the testing kit 20 in the shot image and processing of transmitting the determination result to the user terminal 40, on the basis of the shot image received from the user terminal 40.

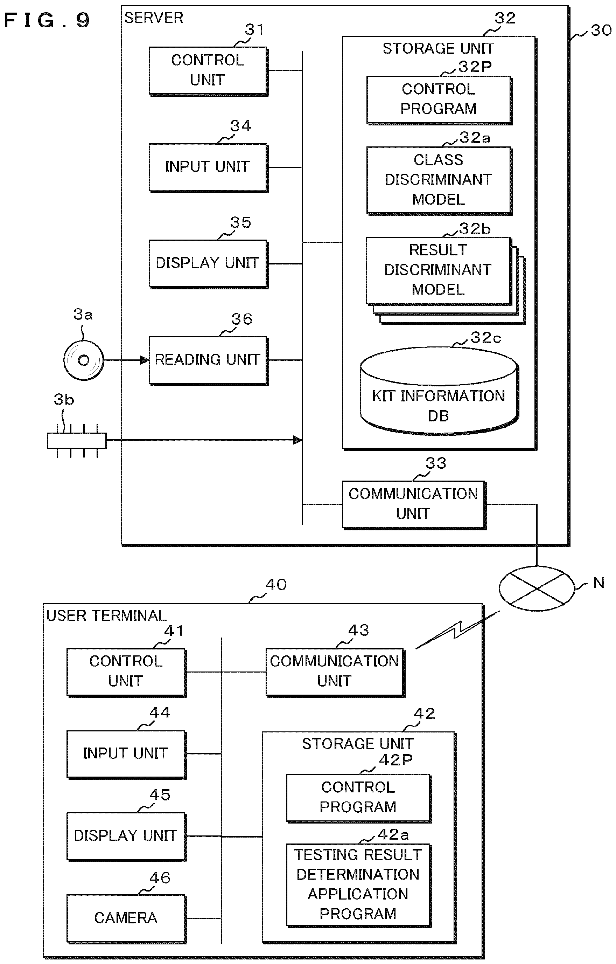

FIG. 9 is a block diagram illustrating a configuration example of the testing system of Embodiment 2. The user terminal 40 includes a control unit 41, a storage unit 42, a communication unit 43, an input unit 44, a display unit 45, a camera 46, and the like, in which such units are connected to each other through a bus. The control unit 41 includes one or a plurality of processors such as a CPU, a MPU, or a GPU. The control unit 41 performs various information processings, control processings, or the like to be performed by the user terminal 40, by suitably executing a control program 42P stored in the storage unit 42.

The storage unit 42 includes a RAM, a flash memory, a hard disk, a SSD, and the like. The storage unit 42 stores in advance the control program 42P executed by the control unit 41, various data items required for executing the control program 42P, and the like. In addition, the storage unit 42 temporarily stores data or the like that is generated when the control unit 41 executes the control program 42P. Further, the storage unit 42 stores a testing result determination application program 42a for inquiring the server 30 about the testing result of the testing kit 20 in the shot image, on the basis of the shot image of the testing kit 20.

The communication unit 43 is an interface to be connected to the network N by wired communication or wireless communication. The input unit 44 receives the manipulation input of the user, and transmits the control signal corresponding to the manipulation content to the control unit 41. The display unit 45 is a liquid crystal display, an organic EL display, or the like, and displays various information items, in accordance with an instruction from the control unit 41. Note that, the input unit 44 and the display unit 45 may be a touch panel in which the input unit 44 and the display unit 45 are integrally configured. The camera 46 performs shooting, in accordance with an instruction from the control unit 41, and sequentially transmits the acquired image data (the shot image) to the control unit 41.

In the user terminal 40, the control unit 41 may download the control program and the data to be stored in the storage unit 42 from an external device through the communication unit 43 and the network N to be stored in the storage unit 42. In addition, in a case where the user terminal 40 includes a reading unit (not illustrated) reading information stored in a portable storage medium, the control unit 41 may read the control program and the data from the portable storage medium through the reading unit to be stored in the storage unit 42.

The server 30 includes a control unit 31, a storage unit 32, a communication unit 33, an input unit 34, a display unit 35, a reading unit 36, and the like, in which such units are connected to each other through a bus. The control unit 31 includes one or a plurality of processors such as a CPU, a MPU, or a GPU. The control unit 31 allows the server 30 to perform various information processings, control processings, or the like to be performed by the information processing device of the present disclosure, by suitably executing a control program 32P stored in the storage unit 32. The storage unit 32 includes a RAM, a flash memory, a hard disk, a SSD, and the like. The storage unit 32 stores in advance the control program 32P executed by the control unit 31, various data items required for executing the control program 32P, and the like. In addition, the storage unit 32 temporarily stores data or the like that is generated when he control unit 31 executes the control program 32P. In addition, the storage unit 32 stores a class discriminant model 32a and a result discriminant model 32b, which are identical to the class discriminant model 12a and the result discriminant model 12b of Embodiment 1, and a kit information DB 32c which is identical to the kit information DB 12c of Embodiment 1. Even in the server 30 of this embodiment, the result discriminant model 32b is provided for each class (type) of the testing kit 20 that is a discriminant target of the class discriminant model 32a. In addition, the kit information DB 32c may be stored in an external storage device that is connected to the server 30, or may be stored in an external storage device to which the server 30 is capable of performing communication through the network N. Note that, in this embodiment, the kit information DB 32c may not be stored in the storage unit 32 of the server 30.

The communication unit 33 is an interface to be connected to the network N by wired communication or wireless communication. The input unit 34 receives the manipulation input of the user, and transmits the control signal corresponding to the manipulation content to the control unit 31. The display unit 35 is a liquid crystal display, an organic EL display, or the like, and displays various information items, in accordance with an instruction from the control unit 31. Note that, the input unit 34 and the display unit 35 may be a touch panel in which the input unit 34 and the display unit 35 are integrally configured. The reading unit 36 reads information stored in a portable storage medium 3a. The control unit 31 may read the control program and the data to be stored in the storage unit 32 from the portable storage medium 3a through the reading unit 36 to be stored in the storage unit 32. In addition, the control unit 31 may download the control program and the data from an external device through the communication unit 33 and the network N to be stored in the storage unit 32. Further, the control unit 31 may read out the control program and the data from a semiconductor memory 3b.

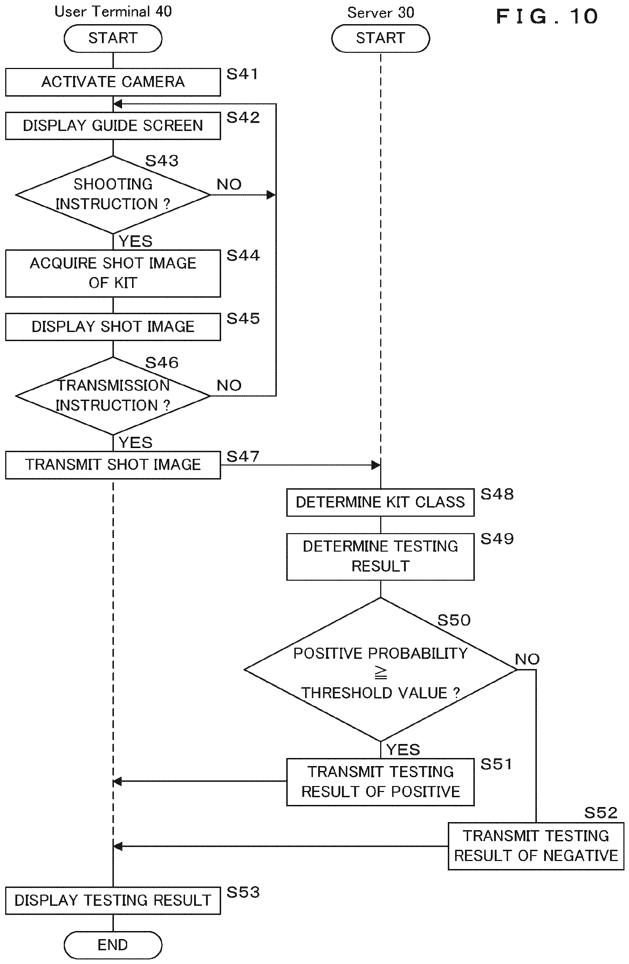

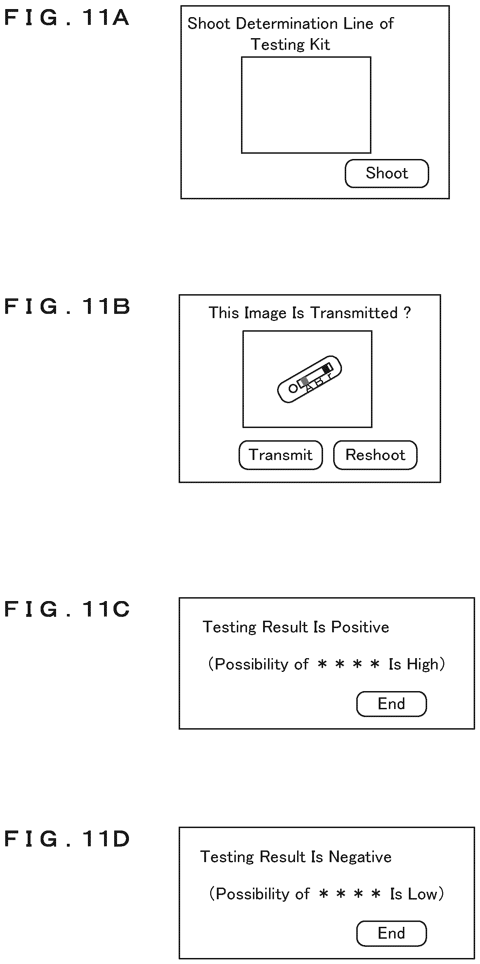

FIG. 10 is a flowchart illustrating an example of a determination processing procedure of the server 30 of Embodiment 2, and FIG. 11A to FIG. 11D are schematic views illustrating a screen example. In FIG. 10, the processing performed by the user terminal 40 is illustrated on the left side, and the processing performed by the server 30 is illustrated on the right side. In this embodiment, the user (the subject) who desires to perform the testing using the testing kit 20, for example, samples a specimen at home, and drops the specimen into the dropping region 21 of the testing kit 20. For example, the subject itself, the family of the subject, or the like (hereinafter, referred to as the user) samples the specimen such as the nasal cavity swab, the pharynx swab, the nasal discharge, the urine, and the saliva of the subject, and drops the specimen into the dropping region 21 of the testing kit 20. Then, after the user, for example, waits for a testing time (the time required for the testing) described in the usage instructions of the testing kit 20 or after the testing end line appears in the determination region 22 of the testing kit 20, the testing result determination application program 42a is activated by using the user terminal 40. Note that, in a case where the testing time is not known or in a case where the appearance of the testing end line is not found, the user may activate the testing result determination application program 42a at an arbitrary timing after the specimen is dropped into the dropping region 21. In a case where the testing result determination application program 42a is executed, the control unit 41 of the user terminal 40 activates the camera 46 (S41) to start the shooting of the moving image using the camera 46.

The control unit 41, for example, displays a guide screen as illustrated in FIG. 11A on the display unit 45 (S42). The guide screen displays a message for guiding the shooting (the shooting of a still image) of the determination region 22 (the determination line and the testing end line) of the testing kit 20. In addition, the guide screen includes an image section for displaying the shot image (a moving image and a still image) of the camera 46 and a shoot button for instructing the shooting of the still image. The control unit 41 sequentially displays the moving image acquired by the camera 46 in the image section on the guide screen, and the user checks the moving image displayed in the image section and manipulates the shoot button through the input unit 44 in a state where the upper surface of the testing kit 20 can be shot. In a case where the shoot button is manipulated on the guide screen, the control unit 41 receives a shooting instruction of the still image using the camera 46, and acquires the shot image (the still image) of the upper surface of the testing kit 20. Accordingly, the control unit 41 judges whether or not the shooting instruction of the still image is received through the guide screen (S43), and in a case where it is judged that the shooting instruction is not received (S43: NO), the control unit 41 continuously displays the guide screen.

In a case where it is judged that the shooting instruction of the still image is received (S43: YES), the control unit 41 performs the shooting of the still image using the camera 46, and acquires the shot image of the upper surface of the testing kit 20 (S44). As illustrated in FIG. 11B, the control unit 41 displays the acquired shot image (still image) in the image section on the guide screen (S45). The guide screen illustrated in FIG. 11B displays the shot image (the still image) of the testing kit 20, and displays a message for inquiring whether or not to transmit the displayed shot image to the server 30. In addition, the guide screen includes a transmit button for instructing the transmission of the shot image and a reshoot button for instructing the reshooting of the shot image of the testing kit 20. In a case where the shot image displayed in the image section is transmitted to the server 30, the user manipulates the transmit button through the input unit 44 to instruct the transmission of the shot image to the server 30, and in a case where the shot image is reshot, the user manipulates the reshoot button through the input unit 44 to instruct the reshooting of the shot image. Accordingly, the control unit 41 judges whether or not the a transmission instruction of the shot image is received through the guide screen (S46), and in a case where it is judged that the transmission instruction is not received (S46: NO), that is, in a case where the reshooting is instructed, the process returns to the processing of step S42, and returns to the display of the guide screen illustrated in FIG. 11A. In a case where it is judged that the transmission instruction of the shot image is received (S46: YES), the control unit 41 transmits the shot image being displayed in the image section to the server 30 (S47). Note that, the control unit 41 transmits information (for example, address information) of the own user terminal 40 to the server 30 together with the shot image.

The control unit 31 (an image acquisition unit) of the server 30 receives the shot image of the testing kit 20 from the user terminal 40, and determines the class of the testing kit 20 in the received shot image by using the class discriminant model 32a (S48). Here, as with step S17 in FIG. 5, the control unit 31 inputs the shot image to the class discriminant model 32a, and specifies the class of the testing kit 20 in the shot image, on the basis of output information from the class discriminant model 32a. In a case where the class of the testing kit 20 is specified, the control unit 31 (a determination unit) specifies the result discriminant model 32b according to the specified class, and determines the testing result of the testing kit 20 in the received shot image by using the specified result discriminant model 32b (S49). Here, as with step S19 in FIG. 6, the control unit 31 inputs the shot image to the result discriminant model 32b, and determines whether the testing result of the testing kit 20 in the shot image is positive or negative, on the basis of output information from the result discriminant model 32b. Specifically, the control unit 31 acquires a probability (a positive probability) that the testing result is to be discriminated as a positive.

The control unit 31 judges whether or not the acquired positive probability is greater than or equal to the predetermined threshold value (for example, greater than or equal to 80%) (S50). In a case where it is judged that the positive probability is greater than or equal to the threshold value (S50: YES), the control unit 31 (an output unit) transmits the determined result (here, a positive testing result) to the user terminal 40 (S51). Note that, the control unit 31 transmits the determination result (the testing result) to the user terminal 40 by using the information (the address information) of the user terminal 40 that is received from the user terminal 40 together with the shot image. On the other hand, in a case where it is judged that the acquired positive probability is less than the predetermined threshold value (S50: NO), the control unit 31 (the output unit) transmits a negative testing result to the user terminal 40 (S52).

In a case where the determination result (the testing result) is received from the server 30, the control unit 41 of the user terminal 40 displays the received testing result on the display unit 45 (S53), and ends the processing. FIG. 11C illustrates a display example of the testing result determined by the server 30 as a positive, and FIG. 11D illustrates a display example of the testing result determined by the server 30 as a negative. According to such a notification screen, the user of the user terminal 40 is capable of acquiring the testing result of the testing kit 20 shot by the user itself from the server 30. Accordingly, in this embodiment, in a case where the testing is performed at home by using the testing kit 20, the testing result of the testing kit 20 can be determined by the server 30. Accordingly, it is not necessary for the user to grasp the usage of the testing kit 20, and it is possible for the user to more easily use the testing kit 20. In addition, for example, in a case where a health management application for managing biological data such as the body height, the body weight, the blood pressure, the body temperature, and the heart rate of the user is installed in the user terminal 40, the control unit 41 may link the testing result of the testing kit 20 that is received from the server 30 with the health management application. In this case, since the user is capable of grasping the testing result of the testing kit 20 through the health management application that is familiar to the user, convenience is improved.

In this embodiment, the result discriminant model 32b can also be configured to output a probability (a determination unavailable probability) that it is to be determination unavailable, in addition to a probability (a positive probability) that the testing result is to be discriminated as a positive and a probability (a negative probability) that the testing result is discriminated as a negative. For example, the result discriminant model 32b may be learned to output a high value as the probability that it is to be determination unavailable with respect to the shot image of the testing kit 20 in a state where the testing end line does not appear. In this case, the server 30 may be configured not only to confirm that the testing result is positive or negative, on the basis of the positive probability and the negative probability, but also to request the retransmission of the shot image of the testing kit 20 to the user terminal 40 in a case where the determination unavailable probability is greater than or equal to the predetermined threshold value (for example, greater than or equal to 50%). The user terminal 40 to which the retransmission of the shot image is requested, for example, returns to the display of the guide screen illustrated in FIG. 11A, acquires again the shot image of the testing kit 20, and transmits the shot image to the server 30, and the server 30 is capable of determining again the testing result, on the basis of the received shot image. Accordingly, for example, even in a case where the testing kit 20 is first shot before the elapse of the testing time or even in a case where the shot image is an image in which the testing result is not capable of being determined, the testing result is determined by using the image that is shot again, and an accurate testing result of the testing kit 20 can be notified to the user.

In the user terminal 40 of this embodiment, the still image of the testing kit 20 may be automatically shot, instead of the configuration in which the still image of the testing kit 20 is acquired on the basis of the manipulation of the user. That is, in a case where the moving image of the testing kit 20 is shot by the camera 46, the user terminal 40 may be configured such that the still image of the testing kit 20 is automatically acquired at a timing when a highly accurate still image can be shot. In this case, the user is capable of acquiring the still image of the testing kit 20 with a high accuracy, only by holding the user terminal 40 over the upper surface of the testing kit 20 (only by shooting the moving image of the testing kit 20 with the camera 46). Accordingly, it is not necessary for the user, for example, to manipulate the shoot button on the guide screen illustrated in FIG. 11A, and manipulation properties of the user are further improved.

In this embodiment, the user terminal 40 may be a terminal to be used by the tester in the medical institution or the like, in addition to the terminal of the subject, the family of the subject, or the like. For example, one server 30 can be provided in one medical institution, and a tester in the medical institution is capable of shooting the testing kit 20 by using the user terminal 40, transmitting the shot image to the server 30, and acquiring the determination result from the server 30. In this case, in a large-scale medical institution, even in a case where there are a plurality of places for performing the testing by using the testing kit 20, an accurate testing result of the testing kit 20 can be obtained by acquiring the shot image of the testing kit 20 in each testing place (for example, an examination room, a treatment room, or the like of each diagnosis and treatment department) and transmitting the shot image to the server 30. In addition, the server 30 may be prepared on the network N, and testers in a plurality of medical institutions may shoot the testing kit 20 by using the user terminal 40, transmit the shot image to the server 30, and acquire the determination result from the server 30. In this case, one server 30 can be shared by the testers in the plurality of medical institutions. In addition, even in a case where a new type of testing kit 20 is sold, the class discriminant model 32a and the result discriminant model 32b can be updated on a manager side for managing the server 30. Accordingly, the determination result using the newest class discriminant model 32a and result discriminant model 32b can be obtained on a medical institution side, without performing update processing of the discriminant models 32a and 32b.

### (Embodiment 3)

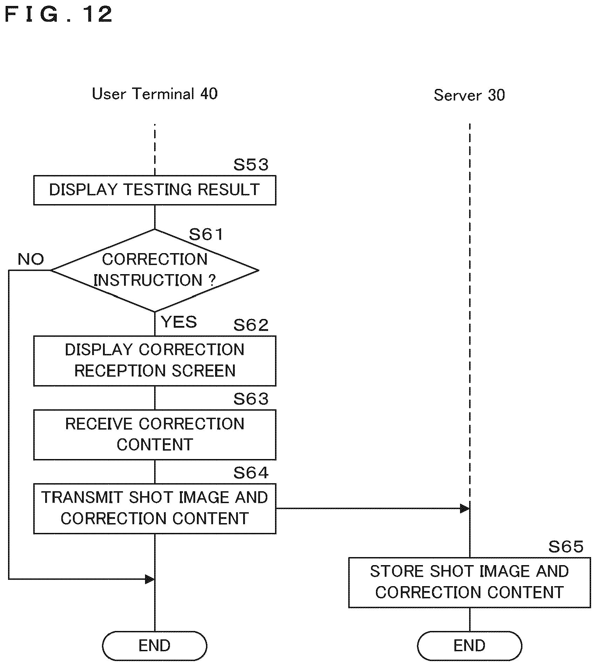

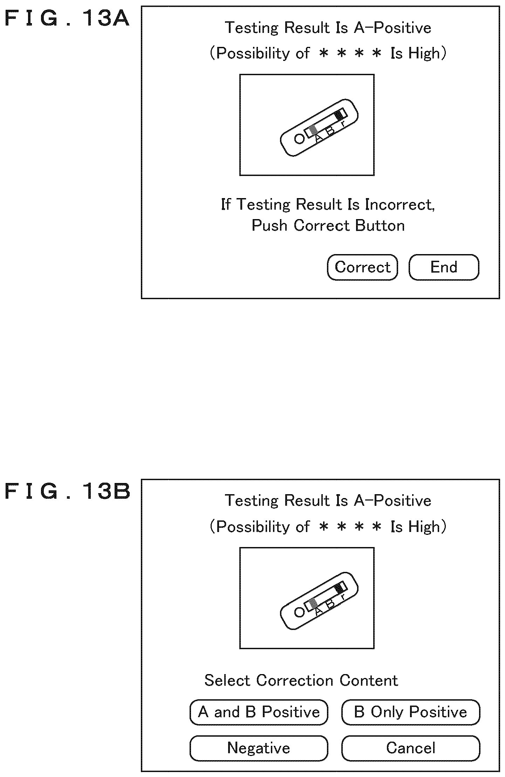

A testing system having a configuration in which the correction content is transmitted to the server 30 from the user terminal 40 in a case where the result (the testing result of the testing kit 20) that is determined by the server 30 is incorrect, in the testing system of Embodiment 2, will be described. Since the testing system of this embodiment is attained by the same device as each of the devices 30 and 40 in the testing system of Embodiment 2, the description of the configuration will be omitted. FIG. 12 is a flowchart illustrating an example of a determination processing procedure of the server 30 of Embodiment 3, and FIG. 13A and FIG. 13B are schematic views illustrating a screen example. The processing illustrated in FIG. 12 further includes steps S61 to S65 after step S53 in the processing illustrated in FIG. 10. The description of the same steps as those in FIG. 10 will be omitted. In addition, in FIG. 12, steps S41 to S52 in FIG. 10 are not illustrated.

In this embodiment, as with Embodiment 2, the control unit 41 of the user terminal 40 performs the processing of steps S41 to S47, and the control unit 31 of the server 30 performs the processing of steps S48 to S52. Accordingly, the server 30 determines the testing result of the testing kit 20, on the basis of the shot image of the testing kit 20 that is shot by the user terminal 40, and transmits the determined result to the user terminal 40. Then, the control unit 41 of the user terminal 40 displays the determination result (the testing result) that is received from the server 30 on the display unit 45 (S53). In the server 30 of this embodiment, in a case where the testing result of the testing kit 20 is determined as a positive, specifically, in a case where it is determined that the positive probability is greater than or equal to the predetermined threshold value, the control unit 31 generates a testing result screen as illustrated in FIG. 13A to be transmitted to the user terminal 40. Accordingly, the user terminal 40 displays the testing result screen as illustrated in FIG. 13A on the display unit 45. The testing result screen illustrated in FIG. 13A displays a message for notifying that the testing result of the testing kit 20 is determined as a positive and the shot image of the testing kit 20 that is used for determining the testing result. Note that, in a case where the testing result of the testing kit 20 is determined as a negative, the control unit 31 of the server 30 generates a testing result screen for notifying that the testing result is determined as a negative to be transmitted to the user terminal 40.

In addition, the testing result screen includes a correct button for instructing the correction (the modification) of the notified determination result and an end button for instructing the end of the testing result determination application program 42a. Accordingly, the user visually determines the testing result of the determination region 22 (the determination line) of the testing kit 20 used for the testing, and judges whether or not the own determination is coincident with the determination result notified by the testing result screen. Then, the user manipulates the end button in a case where the own determination is coincident with the notified determination result, and manipulates the correct button in a case where the own determination is not coincident with the notified determination result. The control unit 41 receives a correction instruction for the notified determination result in a case where the correct button is manipulated on the testing result screen through the input unit 44, and receives an end instruction of the testing result determination application program 42a in a case where the end button is manipulated.

The control unit 41 judges whether or not the correction instruction for the determination result is received (S61), and in a case where it is judged that the correction instruction is received (S61: YES), for example, the control unit 41 displays a correction reception screen as illustrated in FIG. 13B on the display unit 45 (S62). For example, in a case where the determination result is positive in the influenza A testing, as illustrated in FIG. 13B, the correction reception screen displays each button (an influenza A and influenza B-both-positive button, a negative button, and an influenza B-only-positive button) for designating the testing result that is capable of being corrected with respect to the determination result (the correction content) and a cancel button for ending the correction processing. Accordingly, in a case where the determination result is corrected, the user manipulates the button corresponding to the content to be corrected, and in a case where the determination result is not corrected, the user manipulates the cancel button.

In a case where the cancel button is manipulated on the correction reception screen through the input unit 44, the control unit 41 ends the display of the correction reception screen and returns to the display of the testing result screen. In a case where the button corresponding to the correction content is manipulated on the correction reception screen, the control unit 41 receives the correction content corresponding to the manipulated button (S63). That is, for example, in a case where the influenza A and influenza B-both-positive button is manipulated, the control unit 41 receives a correction instruction for positive in both of the influenza A testing and the influenza B testing. The control unit 41 associates the received correction content with the shot image of the testing kit 20 displayed on the testing result screen to be transmitted to the server 30 (S64).

In a case where the correction content and the shot image are received from the user terminal 40, the control unit 31 of the server 30 associates the received correction content with the shot image, for example, to be stored in the storage unit 32 (S65), and ends the processing. Note that, in a case where it is judged that the correction instruction for the determination result is not received through the testing result screen (S61: NO), that is, in a case where the end instruction of the testing result determination application program 42a is received, the control unit 41 ends the processing by skipping the processing of steps S62 to S64. The correction content and the shot image, which are stored in the server 30, can be used in teacher data (relearning data) for relearning the result discriminant model 32b corresponding to the class of the testing kit 20 in the shot image. For example, the control unit 31 of the server 30 determines the class of the testing kit 20 in the shot image of the relearning data by using the class discriminant model 32a, and is capable of relearning the result discriminant model 32b corresponding to the determined class by using the shot image of the relearning data and the testing result indicated by the correction content as the teacher data. Accordingly, it is possible to obtain the result discriminant model 32b that is capable of discriminating the testing result of the testing kit 20 with a higher accuracy.

In this embodiment, the same effect as that in Embodiment 2 can also be obtained. In addition, in this embodiment, in a case where the testing result (the determination result) that is determined by the server 30 is incorrect, the server 30 is capable of acquiring a correct testing result from the user terminal 40. Accordingly, the server 30 is capable of acquiring the correct testing result and the shot image of the testing kit 20 from the user terminal 40, and collecting teacher data for relearning the result discriminant model 32b. In addition, in a case where the result discriminant model 32b is relearned by using the collected teacher data, a more accurate result discriminant model 32b can be attained.

### (Embodiment 4)

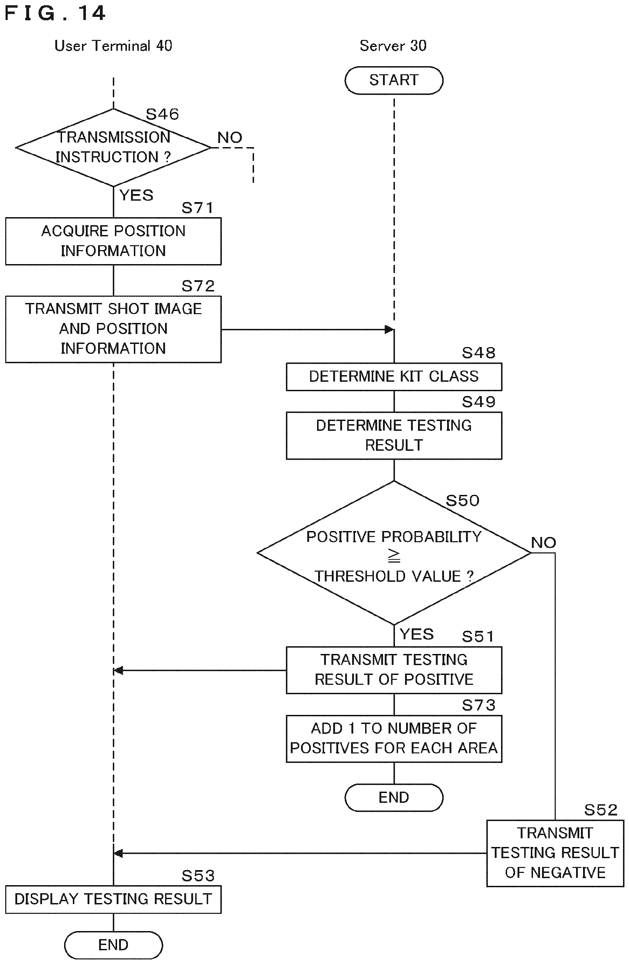

A testing system having a configuration in which position information indicating the current position of the user terminal 40 is also transmitted when the user terminal 40 transmits the shot image of the testing kit 20 to the server 30, in the testing system of Embodiment 2, will be described. Since the testing system of this embodiment is attained by the same device as each of the devices 30 and 40 in the testing system of Embodiment 2, the description of the configuration will be omitted. FIG. 14 is a flowchart illustrating an example of a determination processing procedure of the server 30 of Embodiment 4. The processing illustrated in FIG. 14 includes steps S71 to S72 instead of step S47 in the processing illustrated in FIG. 10, and further includes steps S73 after step S51. The description of the same steps as those in FIG. 10 will be omitted. In addition, in FIG. 14, steps S41 to S45 in FIG. 10 are not illustrated.

In this embodiment, as with Embodiment 2, the control unit 41 of the user terminal 40 performs the processing of steps S41 to S46. Then, in a case where it is judged that the transmission instruction of the shot image is received through the guide screen (S46: YES), the control unit 41 of the user terminal 40 acquires position information indicating the current position of the own user terminal 40 (S71). For example, in a case where the user terminal 40 includes a GPS antenna receiving an electric wave to be transmitted from a global positioning system (GPS) satellite, the control unit 41 acquires the current position, on the basis of the electric wave received through the GPS antenna. In addition, for example, the control unit 41 may acquire the position information by the input from the user through the input unit 44. In this case, the control unit 41, for example, may be configured to display a select screen for selecting any of the prefectures in Japan or any of the districts such as the Tohoku district, the Kanto district, and the Kinki district on the display unit 45, and receive the selection of the position information through the select screen. Note that, an acquisition method of the current position is not limited to such methods. The control unit 41 associates the acquired position information with the shot image of the shot image being displayed on the guide screen to be transmitted to the server 30 (S72).

The control unit 31 (a position acquisition unit) of the server 30 receives the position information and the shot image from the user terminal 40, and performs the processing of steps S48 to S52, on the basis of the received shot image. Accordingly, the testing result determined by the server 30 on the basis of the shot image of the testing kit 20 that is shot by the user terminal 40 can be transmitted to the user terminal 40. Then, the control unit 41 of the user terminal 40 displays the determination result (the testing result) that is received from the server 30 (S53).

In the server 30 of this embodiment, the control unit 31 (a counting unit) specifies an area including a position indicating the position information, on the basis of the position information received from the user terminal 40, after the processing of step S51, and adds 1 to the number of positives corresponding to the specified area (S73). That is, in a case where a positive testing result is obtained, the server 30 counts the number of positives (an incidence) for each area (each position information item) of the user terminal 40. Note that, the area, for example, may be the prefectures in Japan, or may be the districts such as the Tohoku district, the Kanto district, and the Kinki district. As described above, the server 30 counts the incidence (the number of positives) for each of the areas, and thus, it is possible to predict the trend of pandemic of diseases such as influenza, and the like. The control unit 31 stores the number of positives respectively corresponding to a plurality of areas set in advance, for example, in the storage unit 32, and counts the number of positives for each of the areas by adding 1 to the number of positives of each of the areas that is stored in the storage unit 32. Note that, the control unit 31 may count the number of positives of each of the areas for each date, or may count the number of positives of each of the areas for each predetermined time zone in one day.

In this embodiment, the same effect as that in Embodiment 2 can also be obtained. In addition, in this embodiment, the server 30 is capable of counting the number of times of determining the testing result as a positive (the number of positives), on the basis of the shot image received from the user terminal 40, for each of the areas. In addition, the configuration of this embodiment can also be applied to the testing system of Embodiment 3, and even in a case where the configuration is applied to the testing system of Embodiment 3, the same effect can be obtained.

### (Embodiment 5)

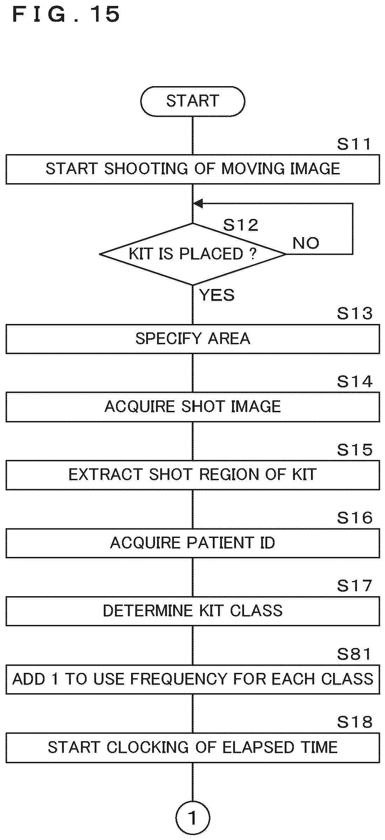

The testing device 10 having a configuration in which the use frequency of the testing kit 20 is counted for each class of the testing kit 20, in the testing device 10 of Embodiment 1, will be described. Since the testing device of this embodiment has the same configuration as that of the testing device 10 of Embodiment 1, the description of the configuration will be omitted. FIG. 15 is a flowchart illustrating an example of a determination processing procedure of the testing device 10 of Embodiment 5. The processing illustrated in FIG. 15 further includes step S81 between step S17 and step S18, in the processing illustrated in FIG. 5 and FIG. 6. The description of the same steps as those in FIG. 5 and FIG. 6 will be omitted. In addition, in FIG. 15, each step in FIG. 6 is not illustrated.

In this embodiment, as with Embodiment 1, the control unit 11 of the testing device 10 performs the processing of steps S11 to S17. Then, in the testing device 10 of this embodiment, the control unit 11 adds 1 to the use frequency of the testing kit 20 corresponding to the class of the testing kit 20 that is determined in step S17 (S81). Accordingly, the use frequency of the testing kit 20 of which the testing result is determined by the testing device 10 can be counted for each of the classes. Note that, the control unit 11 stores the use frequency corresponding to each of the classes of the testing kit 20, for example, in the storage unit 12, and counts the use frequency for each of the classes by adding 1 to the use frequency of each of the classes that is stored in the storage unit 12. In addition, the control unit 11 may count the use frequency of each of the classes for each date, or may count the use frequency of each of the classes for each predetermined period such as one week or one month. As with Embodiment 1, the control unit 11 performs processing subsequent to step S18 after the processing of step S81.

In this embodiment, the same effect as that in Embodiment 1 can also be obtained. In addition, in this embodiment, for the testing kit 20 of which the testing result is determined by the testing device 10, the use frequency for each of the classes can be counted. Accordingly, since the actual use frequency for the testing kit 20 of each of the classes can be grasped, stock management of the testing kit 20 is facilitated, and the number of stocks to be purchased next is easily determined. In addition, since the stock quantity of each of the testing kits 20 can be grasped, for example, in a case where the stock quantity is less than a predetermined number, a function of automatically passing an order to a manufacturer that is a distribution source can also be provided in the testing device 10.

The configuration of this embodiment can also be applied to the testing systems of Embodiments 2 to 4. Note that, in a case where the configuration is applied to the testing systems of Embodiments 2 to 4, for example, for the testing kit 20 that is used by the subject at home, the use frequency for each of the classes can be counted. Accordingly, for example, it is possible to grasp sharing between the manufacturers. In addition, each of the manufacturers is capable of suitably determining a manufacturing number of the testing kits 20 to be manufactured next, on the basis of use frequency for each of the classes, and suppressing the occurrence of excessive stocks.

### (Embodiment 6)

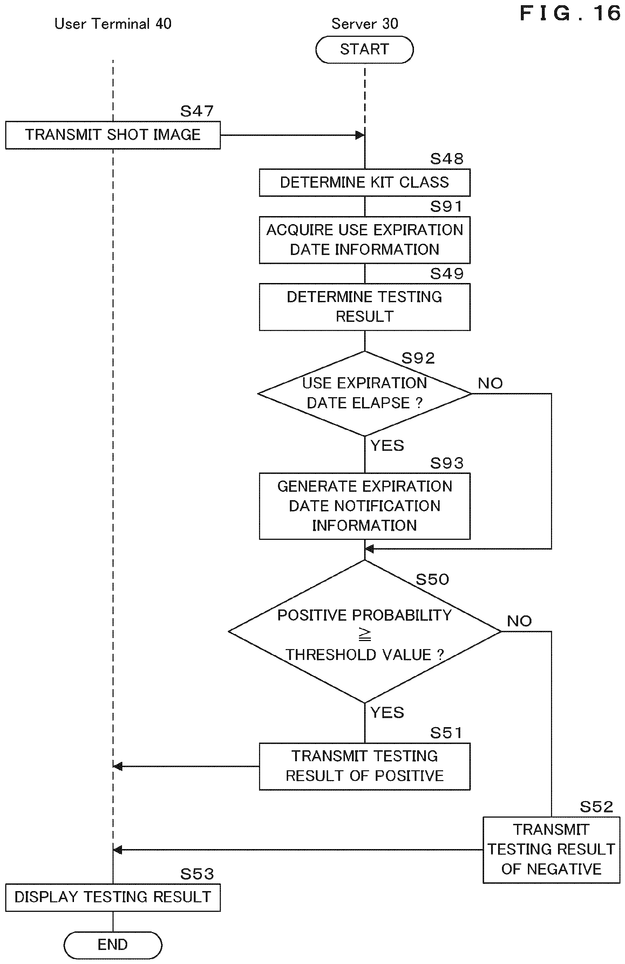

A testing system having a configuration in which information relevant to a use expiration date of the testing kit 20 in the shot image is acquired on the basis of the shot image received by the server 30 from the user terminal 40, and in a case where a use expiration date elapses, that effect is notified to the user of the user terminal 40, in the testing system of Embodiment 2, will be described. Since the testing system of this embodiment is attained by the same device as each of the devices 30 and 40 of the testing system of Embodiment 2, the description of the configuration will be omitted. FIG. 16 is a flowchart illustrating an example of a determination processing procedure of the server 30 of Embodiment 6. The processing illustrated in FIG. 16 further includes step S91 between step S48 and step S49, and further includes steps S92 and S93 between step S49 and step S50, in the processing illustrated in FIG. 10. The description of the same steps as those in FIG. 10 will be omitted. In addition, in FIG. 16, steps S41 to S46 in FIG. 10 are not illustrated.

In this embodiment, as with Embodiment 2, the control unit 41 of the user terminal 40 performs the processing of steps S41 to S47, and the control unit 31 of the server 30 performs the processing of step S48. Then, in the server 30 of this embodiment, the control unit 31 (an expiration date acquisition unit) acquires information (use expiration date information) relevant to the use expiration date of the testing kit 20 in the shot image from the shot image received from the user terminal 40 (S91). For example, the use expiration date information (year-month-day of the use expiration date) may be printed on the upper surface of each of the testing kits 20, and the control unit 31 may read the use expiration date information from the shot image. In addition, in a case where the code 23 of each of the testing kits 20 includes the use expiration date information, the control unit 31 may acquire the use expiration date information from the information of the code 23 that is read from the shot image. In addition, kit information such as a product number or a lot number of the testing kit 20 may be printed on the upper surface of each of the testing kits 20, and the control unit 31 may read the kit information from the shot image or specify the use expiration date corresponding to the read kit information. In this case, information of the use expiration date corresponding to the kit information of the testing kit 20 may be stored in advance in the storage unit 32. In addition, the kit information may be included in the code 23 of each of the testing kits 20, the kit information may be acquired from the information of the code 23 that is read from the shot image, and the information of the use expiration date corresponding to the acquired kit information may be acquired.

The control unit 31 of the server 30 acquires the use expiration date information, and then, performs the processing of step S49, and determines the testing result of the testing kit 20 in the received shot image. Then, the control unit 31 (the judgement unit) judges whether or not the use expiration date elapses, on the basis of the use expiration date information acquired in step S91 (S92). For example, the control unit 31 judges whether or not the use expiration date elapses by comparing the current date and time at this time point with the use expiration date. In addition, the control unit 31 may judge whether or not the use expiration date elapses by comparing a reception date and time when the shot image is received from the user terminal 40 with the use expiration date, and in a case where information of the shooting date is included in the shot image received from the user terminal 40, the control unit 31 may judge whether or not the use expiration date elapses by comparing the shooting date with the use expiration date.

In a case where it is judged that the use expiration date elapses (S92: YES), the control unit 31 generates a message (expiration date notification information) for notifying that the use expiration date elapses (S93). In a case where it is judged that the use expiration date does not elapse (S92: NO), the control unit 31 skips the processing of step S93. Then, the control unit 31 judges whether or not the positive probability acquired in step S49 is greater than or equal to the predetermined threshold value (S50), and in a case where it is judged that the positive probability is greater than or equal to the threshold value (S50: YES), the control unit 31 transmits the positive testing result to the user terminal 40, together with the message (the expiration date notification information) generated in step S93 (S51). In addition, in a case where it is judged that the positive probability is less than the predetermined threshold value (S50: NO), the control unit 31 transmits the negative testing result to the user terminal 40, together with the message (the expiration date notification information) generated in step S93 (S52). Accordingly, in a case where the use expiration date elapses, the control unit 41 of the user terminal 40 displays a message for notifying that effect, together with the determination result (the testing result) of the server 30 (S53). Accordingly, when the testing kit 20 of which the use expiration date has elapsed is accidentally used by the user, the server 30 is capable of notifying that effect to the user (the subject). Accordingly, the user is capable of performing again the testing by using the testing kit 20 of which the use expiration date does not elapse, and obtaining an accurate testing result.

In this embodiment, the same effect as that in Embodiment 2 can also be obtained. In addition, in this embodiment, the server 30 is capable of checking that the use expiration date of the testing kit 20 does not elapse, on the basis of the shot image received from the user terminal 40. In addition, in a case where the use expiration date elapses, the elapse of the use expiration date is notified to the user terminal 40 from the server 30, and thus, the user is capable of grasping that the testing kit 20 of which the use expiration date elapses is used. In addition, the configuration of this embodiment can also be applied to the testing systems of Embodiments 3 and 4, and even in a case where the configuration is applied to the testing systems of Embodiments 3 and 4, the same effect can be obtained.

### (Embodiment 7)

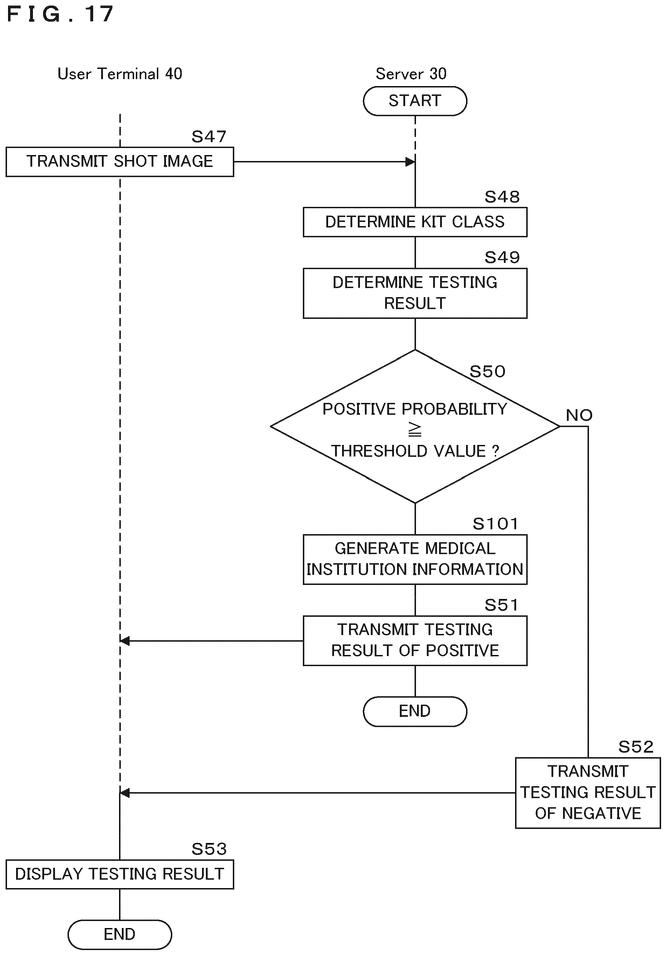

A testing system having a configuration in which in a case where the server 30 determines the testing result of the testing kit 20 in the shot image as a positive, on the basis of the shot image received from the user terminal 40, information of a medical institution in which a treatment can be performed, information of an over-the-counter drug product, or the like is provided to the user of the user terminal 40, in the testing system of Embodiment 2, will be described. Since the testing system of this embodiment is attained by the same device as each of the devices 30 and 40 in the testing system of Embodiment 2, the description of the configuration will be omitted. FIG. 17 is a flowchart illustrating an example of a determination processing procedure of the server 30 of Embodiment 7. The processing illustrated in FIG. 17 further includes step S101 before step S51, in the processing illustrated in FIG. 10. The description of the same steps as those in FIG. 10 will be omitted. In addition, in FIG. 17, steps S41 to S46 in FIG. 10 are not illustrated.

In this embodiment, as with Embodiment 2, the control unit 41 of the user terminal 40 performs the processing of steps S41 to S47, and the control unit 31 of the server 30 performs the processing of steps S48 to S50. Then, in the server 30 of this embodiment, in a case where it is judged that the acquired positive probability is greater than or equal to the predetermined threshold value (S50: YES), the control unit 31 generates the information (medical institution information and introduction information) of the medical institution or the diagnosis and treatment department in which a treatment can be performed, corresponding to a disease that is determined as a positive (S101). For example, the information of the medical institution or the diagnosis and treatment department in which each of the diseases can be treated is stored in the storage unit 32 of the server 30, corresponding to the class of the testing kit 20 or the testing target of the testing kit 20. Then, the control unit 31 reads out the information of the medical institution or the diagnosis and treatment department corresponding to the class of the testing kit 20 that is determined in step S48 from the storage unit 32. Then, the control unit 31 transmits the read information of the medical institution or the diagnosis and treatment department, and the determined testing result (here, a positive testing result) together to the user terminal 40 (S51). Accordingly, the control unit 41 of the user terminal 40 displays the information of the medical institution or the diagnosis and treatment department in which a treatment can be performed, together with the determination result (the testing result) of the server 30 (S53). Note that, the information to be provided to the user of the user terminal 40 may include the information of the over-the-counter drug product or the pharmacy, in addition to the information of the medical institution or the diagnosis and treatment department.

In this embodiment, the same effect as that in Embodiment 2 can also be obtained. In addition, in this embodiment, in a case where the server 30 determines the testing result as a positive, on the basis of the shot image received from the user terminal 40, the information of the medical institution or the diagnosis and treatment department, the information of the over-the-counter drug product or the pharmacy, or the like can be provided to the user (the subject) to propose visiting to the medical institution or taking the over-the-counter drug product. In addition, the configuration of this embodiment can also be applied to the testing systems of Embodiments 3, 4, and 6, and even in a case where the configuration is applied to the testing systems of Embodiments 3, 4, and 6, the same effect can be obtained.

### (Embodiment 8)

A configuration in which the output node that outputs the probability that it is to be determined that it is not possible to discriminate which class of the discriminant target the class of the testing kit 20 in the shot image is (discrimination unavailable) is provided on the output layer of the class discriminant model 12a, in the testing device 10 of Embodiment 1, will be described. Since the testing device 10 of this embodiment has the same configuration as that of the testing device 10 of Embodiment 1, except for the class discriminant model 12a, the description of the configuration will be omitted. Note that, the class discriminant model 12a of this embodiment is configured to discriminate which class of the testing kit 20 that is learned as the discriminant target the class of the testing kit 20 in the shot image is or it is not possible to discriminate which class of the testing kit 20 that is learned as the discriminant target the class of the testing kit 20 in the shot image is (discrimination unavailable). Accordingly, in the class discriminant model 12a, the output layer includes nodes of which the number is a value obtained by adding 1 to the number of classes of the discriminant target, and one node outputs the probability that the class of the testing kit 20 in the input shot image is to be determined as other than the class of the discriminant target.

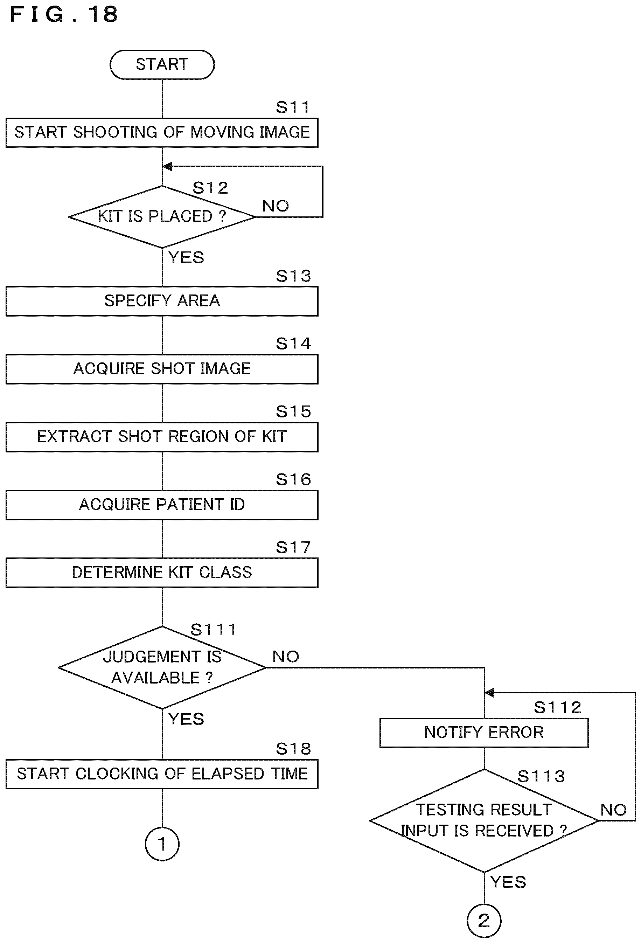

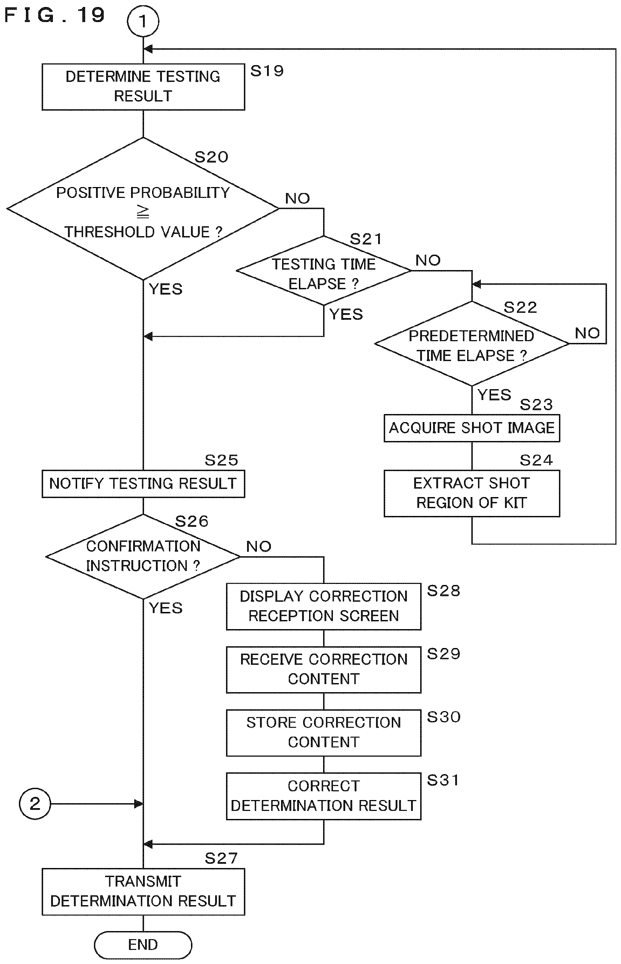

FIG. 18 and FIG. 19 are flowcharts illustrating an example of a determination processing procedure of the testing device 10 of Embodiment 8. The processing illustrated in FIG. 18 and FIG. 19 further includes steps S111 to S113 between step S17 and step S18, in the processing illustrated in FIG. 5 and FIG. 6. The description of the same steps as those in FIG. 5 and FIG. 6 will be omitted. In this embodiment, the control unit 11 of the testing device 10 performs the processing of steps S11 to S17, as with Embodiment 1. Note that, the class discriminant model 12a of this embodiment discriminates which class of the discriminant target the class of the testing kit 20 in the shot image is or the discrimination is unavailable. The control unit 11 judges whether or not it is possible to discriminate which class of the discriminant target the class of the testing kit 20 in the shot image is (determination available), on the basis of the output information from the class discriminant model 12a (Sill). In a case where it is judged that the determination is available (S111: YES), the control unit 11 performs the processing subsequent to step S18.

In a case where it is judged that the determination is unavailable (S111: NO), the control unit 11 displays an error message indicating that the determination is unavailable on the display unit 15, and performs error notification (S112). For example, the control unit 11 displays an error message such as "Class Determination Unavailable" in the testing result screen displayed on the display unit 15, as the status information of the display region corresponding to the testing kit 20 that is the determination target. In addition, the control unit 11 may not only display the error message but also notify the occurrence of an error by the lighting or blinking of the notification light 16, and in a case where the testing device 10 includes the sound output device, the control unit 11 may notify the occurrence of an error by the sound output of the sound output device.

For the testing kit 20 of which the class is not capable of being discriminated, for example, the tester visually checks the testing result, and inputs the testing result through the input unit 14. Accordingly, the control unit 11 judges whether or not the input of the testing result is received with respect to the testing kit 20 of which the error is notified (S113), and in a case where it is judged that the input is not received (S113: NO), the control unit 11 continues the error notification (S112). Note that, for example, in a case where the end of the error notification is instructed through the input unit 14, the control unit 11 may end the error notification. In a case where it is judged that the input of the testing result is received (S113: YES), the control unit 11 proceeds the process to the processing of step S27, transmits the input testing result to a predetermined device (for example, the electronic health record system) (S27), and ends the processing with respect to the area.

In this embodiment, the same effect as that in Embodiment 1 can also be obtained. In addition, in this embodiment, for the testing kit 20 of which the class is not capable of being discriminated by the class discriminant model 12a, the testing result can be inputted by the manual input of the tester, and the testing result can be registered in the electronic health record system. As the testing kit 20, a new class of kit is sold every day. In such a situation, testing result of the testing kit 20 that has been already learned by the class discriminant model 12a can be automatically determined, and the testing result of the testing kit 20 that has not been learned is visually determined by the tester. Accordingly, the tester may visually determine the testing result only of the testing kit 20 of which the class is not been learned by the class discriminant model 12a, and a burden on the tester at the time of performing the testing can be reduced.

The configuration of this embodiment can also be applied to the testing device 10 of Embodiment 5 and the testing systems of Embodiments 2 to 4, 6, and 7. Note that, in a case where the configuration is applied to the testing systems of Embodiments 2 to 4, 6, and 7, for example, in a case where the server 30 determines the class of the testing kit 20 in the shot image by using the class discriminant model 32a, on the basis of the shot image received from the user terminal 40, and in a case where it is possible to determine which class of the discriminant target the class of the testing kit 20 in the shot image is, the testing result of the testing kit 20 in the shot image is determined by using the result discriminant model 32b of the determined class. On the other hand, in a case where it is not possible to determine which class of the discriminant target the class of the testing kit 20 in the shot image is, the server 30 performs the error notification by transmitting the error message indicating the discrimination is unavailable to the user terminal 40. Accordingly, the server 30 is capable of notifying the user of the user terminal 40 not only that the testing result of the testing kit 20 in the shot image transmitted from the user is positive or negative but also that the discrimination is unavailable.

### (Embodiment 9)

In Embodiments 1 to 8 described above, when the testing is performed by using the testing kit 20, the testing result of the testing kit 20 is accurately determined by the testing device 10 or the server 30. In addition, for example, in the manufacturer, the testing device 10 or the server 30 can also be used in a quality testing performed prior to shipment of the testing kit 20. For example, the testing kit 20 prior to shipment can be shot, the class of the testing kit 20 in the shot image can be determined by using the class discriminant models 12a and 32a, and the testing kit 20 can be discriminated as an accepted product or a rejected product in accordance with whether or not the class can be suitably determined. Accordingly, since the quality testing that was visually performed in the related art is automatically performed by using the testing device 10 or the server 30, the quality testing can be efficiently performed, and the testing kit 20 having a small variation in the quality can be provided.

The embodiments disclosed herein are to be considered as an example in all aspects and not restrictive. The scope of the present disclosure is indicated by the scope of claims, but not the meaning described above, and is intended to include all modifications within the meaning and scope equivalent to the scope of claims.

### [Description of Reference Numerals]

- 10: Testing device
- 11: Control unit
- 15: Display unit
- 17: Camera
- 20: Testing kit
- 30: Server
- 31: Control unit
- 33: Communication unit
- 40: User terminal
- 41: Control unit
- 10b: Placing surface
- 12a, 32a: Class discriminant model
- 12b, 32b: Result discriminant model

## Claims

1. A program that causes a computer to execute processing of:
acquiring an image obtained by shooting a testing kit for testing a specimen by using an immunochromatography method;
inputting the acquired image to a learned model for result discrimination that is deep-learned to output information relevant to a testing result of the testing kit in the shot image when the shot image of the testing kit is inputted;
determining the testing result of the testing kit in the acquired image, on the basis of the information outputted from the learned model for result discrimination; and
outputting the determined testing result.

2. The program according to claim 1, causing the computer to execute processing of:
inputting the acquired image to a learned model for class discrimination that is deep-learned to output information relevant to a class of the testing kit in the shot image when the shot image of the testing kit is inputted; and
determining the class of the testing kit in the acquired image, on the basis of the information outputted from the learned model for class discrimination.

3. The program according to claim 2,
wherein the learned model for result discrimination is prepared for each class of the testing kit,
the program causing the computer to execute processing of:
determining the testing result of the testing kit in the acquired image by using the learned model for result discrimination according to the determined class of the testing kit.

4. The program according to claim 2 or 3, causing the computer to execute processing of:
specifying a testing time of the testing kit, in accordance with the determined class of the testing kit;
clocking a remaining time, on the basis of the specified testing time and an elapsed time from a testing start; and
outputting display information displaying the clocked remaining time to a display unit.

5. The program according to any one of claims 1 to 4, causing the computer to execute processing of:
acquiring code information added to the testing kit in the image, on the basis of the acquired image; and
associating the acquired code information with the determined testing result.

6. The program according to any one of claims 1 to 5, causing the computer to execute processing of:
receiving a modification instruction for the determined testing result; and
outputting relearning data including the received modification content and the acquired image.

7. The program according to any one of claims 1 to 6, causing the computer to execute processing of:
acquiring an image obtained by shooting a plurality of testing kits;
extracting a region including each of the testing kits from the acquired image;
determining a testing result by inputting the extracted region to the learned model for result discrimination; and
outputting display information displaying the testing result determined with respect to each region in association with a position of each region in the image to the display unit.

8. The program according to claim 7, causing the computer to execute processing of:
acquiring the image obtained by shooting the plurality of testing kits for each predetermined time;
determining a testing result by inputting each region extracted from the acquired image to the learned model for result discrimination for each predetermined time;
judging whether or not a determination probability of the determined testing result is greater than or equal to a predetermined threshold value; and
outputting display information displaying the determined testing result to the display unit at a time point when it is judged that the determination probability is greater than or equal to the predetermined threshold value.

9. A testing device, comprising:
a placing table on which a plurality of testing kits for testing a specimen by using an immunochromatography method are capable of being placed in parallel;
a shooting unit shooting the testing kits placed on the placing table;
an extraction unit extracting a region including each of the testing kits from the image shot by the shooting unit;
a determination unit inputting the extracted region to a learned model for result discrimination that is deep-learned to output information relevant to a testing result of the testing kit in the shot image when the shot image of the testing kit is inputted and determining the testing result of the testing kit in the extracted region, on the basis of the information outputted from the learned model for result discrimination; and
a display unit displaying the testing result determined with respect to each region in association with a placed position of each of the testing kits placed on the placing table.

10. An information processing apparatus, comprising:
an image acquisition unit acquiring an image obtained by shooting a testing kit for testing a specimen by using an immunochromatography method from a user terminal;
a determination unit inputting the acquired image to a learned model for result discrimination that is deep-learned to output information relevant to a testing result of the testing kit in the shot image when the shot image of the testing kit is inputted and determining the testing result of the testing kit in the acquired image, on the basis of the information outputted from the learned model for result discrimination; and
an output unit outputting the determined testing result to the user terminal.

11. The information processing apparatus according to claim 10, comprising:
a position acquisition unit acquiring position information of the user terminal from the user terminal; and
a counting unit counting the number of images in which the specimen is detected for each area, on the basis of the position information of the user terminal relevant to an image in which the testing result is obtained when the determined testing result indicates detection of the specimen.

12. The information processing apparatus according to claim 10 or 11, comprising:
an expiration date acquisition unit acquiring use expiration date information added to the testing kit in the image, on the basis of the acquired image; and
a judgement unit judging whether or not a use expiration date elapses, on the basis of the acquired use expiration date information,
wherein when it is judged that the use expiration date elapses, the output unit outputs notification information indicating that the use expiration date elapses to the user terminal.

13. The information processing apparatus according to any one of claims 10 to 12,
wherein the output unit outputs introduction information of a medical institution to the user terminal, in accordance with the determined testing result.

14. An information processing method in which a computer executes processing of:
acquiring an image obtained by shooting a testing kit for testing a specimen by using an immunochromatography method;
inputting the acquired image to a learned model for result discrimination that is deep-learned to output information relevant to a testing result of the testing kit in the shot image when the shot image of the testing kit is inputted;
determining the testing result of the testing kit in the acquired image, on the basis of the information outputted from the learned model for result discrimination; and
outputting the determined testing result.
